# EUROPÄISCHE PATENTSCHRIFT

(11) **EP 1 341 742 B1**
(45) Veröffentlichungstag und Bekanntmachung des Hinweises auf die Patenterteilung: **25.05.2005**
(21) Anmeldenummer: 01270510.9
(22) Anmeldetag: 26.11.2001
(51) Int. Cl.: C07B 41/04, C07C 323/15, C07D 339/08, C07C 327/36, C07D 409/08, C07C 49/753, C07D 317/72, C07C 49/467

(54) **VERFAHREN ZUR HERSTELLUNG VON VERBINDUNGEN MIT EINER CF2O-BRÜCKE**
METHOD FOR PRODUCING COMPOUNDS HAVING A CF2O BRIDGE
PROCEDE DE PRODUCTION DE COMPOSES COMPORTANT UN PONT CF2O

(30) Priorität: 12.12.2000 DE 10061790; 23.12.2000 DE 10064996; 02.02.2001 DE 10105313
(43) Veröffentlichungstag der Anmeldung: 10.09.2003
(73) Patentinhaber: MERCK PATENT GmbH, 64293 Darmstadt (DE)
(72) Erfinder: KIRSCH, Peer, 84342 Seeheim-Jugenheim (DE); TAUGERBECK, Andreas, 64285 Darmstadt (DE); HAHN, Alexander, 65428 Rüsselsheim (DE)
(86) Internationale Anmeldenummer: PCT/EP2001/013721
(87) Internationale Veröffentlichungsnummer: WO 2002/048073

(56) Entgegenhaltungen:
- EP-A- 0 786 445
- EP-A- 0 844 229
- WO-A-01/64667
- HAAS A. ET AL.: "Synthese seiten- kettenfluorierter aromatischer Verbindungen und deren chemische Reaktivität" CHEMISCHE BERICHTE., Bd. 121, Nr. 7, 1988, Seiten 1329-1240, XP002189374 VERLAG CHEMIE GMBH. WEINHEIM., DE ISSN: 0009-2940
- SONDEJ S C ET AL: "Gem-Difluoro compounds: a convenient preparation from ketones and aldehydes by halogen fluoride treatment of 1,3-dithiolanes" JOURNAL OF ORGANIC CHEMISTRY, AMERICAN CHEMICAL SOCIETY. EASTON, US, Bd. 51, 1986, Seiten 3508-3513, XP002085716 ISSN: 0022-3263
- SEEBACH D. ET AL.: "Zur Umpolung der Carbonylreaktivität; Deprotonierung von Ketenthioacetalen zu 1,1-dithiosubsti- tuierten Allyl- und Pentadienyllithium- verbindungen sowie deren Reaktionen mit Elektrophilen" LIEBIGS ANNALEN DER CHEMIE., Bd. 5, 1977, Seiten 811-829, XP002189375 VERLAG CHEMIE GMBH. WEINHEIM., DE ISSN: 0170-2041
- KIRSCH P. ET AL.: "Difluorooxymethylene- bridged liquid crystals: a novel synthesis based on the oxidative alkoxydifluorode- sulfuration of dithianylium salts" ANGEWANDTE CHEMIE. INTERNATIONAL EDITION., Bd. 40, Nr. 8, - 17. April 2001 (2001-04-17) Seiten 1480-1484, XP002189376 VERLAG CHEMIE. WEINHEIM., DE ISSN: 0570-0833

## Beschreibung

Die Erfindung betrifft ein Verfahren zur Herstellung von Verbindungen mit mindestens einer -CF₂-O-Brücke im Molekül. Weiterhin betrifft die Erfindung Ketendithioketale und Bis(alkylthio)carbeniumsalze als Ausgangsverbindungen des erfindungsgemäßen Verfahrens. Ferner werden neue, nach dem erfindungsgemäßen Verfahren erhältliche Verbindungen mit mindestens einer -CF₂-O-Brücke im Molekül aufgezeigt.

Verbindungen mit einer, zwei oder mehreren -CF₂-O-Brücken können vor allem Verwendung finden als Flüssigkristalle, aber auch als Pharmazeutika, Pflanzenschutzmittel oder als Vorstufen für solche Produkte oder für die Herstellung von Polymeren.

Flüssigkristalline Verbindungen mit einer oder mehreren -CF₂-O-Brücken eignen sich vorteilhaft als Komponenten flüssigkristalliner Medien, wie sie in optischen und elektrooptischen Anzeigeelementen, wie TN-, STN- und TFT-LCD, verwendet werden. So werden z.B. in der EP 0 844 229 A1 flüssigkristalline Verbindungen beschrieben, die eine -O-CF₂-Brücke enthalten. Zur Herstellung dieser -O-CF₂-Brücke werden verschiedene Verfahren vorgeschlagen. Nach einem der beschriebenen Verfahren wird zunächst ein aromatisches Halogenid in eine Grignard-Verbindung oder in eine lithiierte Verbindung überführt und dann mit Schwefelkohlenstoff in die Dithiocarbonsäure überführt. Die Dithiocarbonsäure wird mit einem Phenol in Gegenwart eines Alkalimetallhydrids und Jod in einen Thioester überführt. Mit einem Fluorierungsmittel wird aus dem Thioester dann die gewünschte -O-CF₂-Brücke gebildet.

Nach einem anderen Verfahren wird vorgeschlagen, zunächst ein Cyclohexanon mit Hexamethylphosphortriamid und Dibromdifluormethan umzusetzen, um ein Difluorhexylidenderivat zu erhalten. An dieses wird zunächst Brom addiert und dann durch Reaktion mit einem Phenolat unter gleichzeitiger Abspaltung von Bromwasserstoff unter Ausbildung einer -CF₂-O-Brücke verethert.

Nachteil dieser Verfahren ist, dass die Reaktionsgeschwindigkeiten niedrig, die Ausbeuten unzufriedenstellend und die Aufarbeitung und Reinigung des Produkts aufwendig sind.

Eine Aufgabe der Erfindung ist es daher, ein Verfahren zur Herstellung von Verbindungen mit mindestens einer -CF₂-O-Brücke im Molekül zur Verfügung zu stellen, das von einfach zugänglichen Edukten ausgeht, eine Isolierung von Zwischenprodukten nicht erfordert und die Produkte in guten Ausbeuten liefert.

Eine weitere Aufgabe der Erfindung ist es, Ausgangsverbindungen und Zwischenprodukte des erfindungsgemäßen Verfahrens aufzuzeigen sowie vorteilhafte Herstellungsverfahren für die Ausgangsverbindungen zur Verfügung zu stellen.

Ferner liegt der Erfindung die Aufgabe zugrunde, neue, nach dem erfindungsgemäßen Verfahren erhältliche Produkte zu beschreiben.

Die Aufgabe wird mit einem Verfahren gemäß Anspruch 1 gelöst. Die Unteransprüche betreffen vorteilhafte Varianten dieses Verfahrens.

Gegenstand der Erfindung ist damit ein Verfahren der eingangs genannten Art, bei dem
a) an mindestens ein Ketendithioketal eine Säure addiert wird,
b) das erhaltene Bis(alkylthio)carbeniumsalz in Gegenwart einer Base mit mindestens einer mindestens eine Hydroxylgruppe aufweisenden Verbindung umgesetzt wird
c) und anschließend, vorzugsweise in situ, der erhaltene Dithioorthoester mit einem Fluorierungsmittel und einem Oxidationsmittel zur Verbindung mit mindestens einer -CF₂-O-Brücke im Molekül oxidativ fluorodesulfuriert wird.

Ein Vorteil des erfindungsgemäßen Verfahrens ist die leichte Zugänglichkeit der Ketendithioketale als Ausgangsverbindungen. Ferner sind die Ketendithioketale durch ihre in der Regel guten Kristallisationseigenschaften gut in hohen Reinheiten zu erhalten, was insbesondere für die Synthese von hochreinen Flüssigkristallen bedeutend ist.

Ein weiterer Vorteil dieses Verfahrens ist die bereits unter sehr schonenden Bedingungen durchführbare Säureaddition an die Ketendithioketale. Hierdurch sind Alkylbis(alkylthio)carbeniumsalze zugänglich, die empfindliche funktionelle Gruppen, wie Ester, Nitrile oder Ketale, enthalten. Zudem können auch Verbindungen mit zwei oder mehr Ketendithioketal-Funktionen je Molekül zu Verbindungen mit entsprechend zwei oder mehr -CF₂O-Brücken umgesetzt werden.

Ferner erfolgt die oxidative Fluorierung zur eine -CF₂-O-Gruppe enthaltenden Verbindung unter sehr milden, leicht basischen Bedingungen und ist daher im Gegensatz zu den konventionellen Methoden mit einer Vielzahl von ungeschützten funktionellen Gruppen, z.B. einer Nitrilgruppe, kompatibel.

Des weiteren ist es von besonderem Vorteil des erfindungsgemäßen Verfahrens, dass die Umsetzung ausgehend vom Ketendithioketal über das Carbeniumsalz und den Dithioorthoester zum Produkt mit mindestens einer -CF₂O-Brücke in einem Reaktionsgemisch, also ohne Isolierung und Aufreinigung der Zwischenprodukte erfolgen kann. Die hierbei erzielbaren Ausbeuten sind hoch bis sehr hoch.

Ein weiterer Gegenstand der Erfindung sind Ketendithioketale sowie die entsprechenden Bis(alkylthio)carbeniumsalze, insbesondere solche gemäß der Ansprüche 20, 21, 23, 24 und 26. Diese Substanzen eignen sich besonders vorteilhaft als Ausgangsverbindungen zum Einsatz in dem erfindungsgemäßen Verfahren zur Herstellung flüssigkristalliner Verbindungen selbst oder von Verbindungen, die in der Synthese flüssigkristalliner Verbindungen Verwendung finden.

Ferner betrifft ein Gegenstand der Erfindung neue, nach dem erfindungsgemäßen Verfahren erhältliche Verbindungen mit mindestens einer CF₂-O-Brücke im Molekül, insbesondere solche nach den Ansprüchen 22 und 25. Diese Verbindungen stellen selbst neue flüssigkristalline Verbindungen dar oder sind vorteilhaft als Synthesebausteine zur Herstellung flüssigkristalliner Verbindungen verwendbar.

Ein neues Diketon und die hieraus zugänglichen Ketale, insbesondere solche gemäß Anspruch 27, sind ebenfalls ein Erfindungsgegenstand. Das Diketon bzw. die entsprechenden Ketale stellen Ausgangsverbindungen bei der Synthese der genannten Ketendithioketale gemäß bevorzugter Verfahrensvarianten dar.

Nachfolgend werden bevorzugte Varianten des erfndungsgemäßen Verfahrens beschrieben.

Bevorzugt werden Ketendithioketale der Formel II 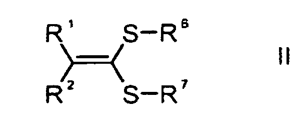 in dem erfindungsgemäßen Verfahren eingesetzt. Hierin bedeuten:
- R¹, R²: unabhängig voneinander geradkettiges, verzweigtes oder cyclisches Alkyl mit 1 bis 25 C-Atomen, wobei R¹, R² derart miteinander verbrückt sein können, dass die Gruppe 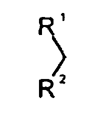 ein cyclisches Alkyl mit 4 bis 8 C-Atomen im Ring ist, vorzugsweise ein Cyclohexan, und/ oder worin ein oder mehrere H-Atome durch Halogen, -CN, weitere gegebenenfalls substituierte Alkyl- und/oder Arylreste ersetzt sein können, und/ oder worin ein oder mehrere nicht benachbarte -CH₂-Gruppen unabhängig voneinander durch -CO-, -O-CO-, -CO-O-, -O-, -S-, -CH=CH-, -C≡C-, -NH- oder -N(CH₃)- ersetzt sein können, und/ oder Aryl, das ein oder mehrfach mit Halogen, geradkettigem, verzweigtem und/ oder cyclischem Alkyl und/ oder Aryl, die ebenfalls substituiert sein können, substituiert sein kann, worin auch ein oder mehrere CH-Gruppen durch N ersetzt sein können, bedeuten,
wobei einer der Reste R¹, R² auch H bedeuten kann,
- R⁶, R⁷: unabhängig voneinander geradkettiges, verzweigtes oder cyclisches Alkyl mit 1 bis 12 C-Atomen, wobei R⁶, R⁷ derart miteinander verbrückt sein können, dass die Gruppe 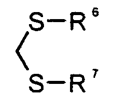 ein 4- bis 8-gliedriger Ring ist, und/ oder worin ein oder mehrere H-Atome durch Halogen, weitere gegebenenfalls substituierte Alkyl- und/ oder Arylreste ersetzt sein können, und/ oder worin ein oder mehrere nicht benachbarte -CH₂-Gruppen unabhängig voneinander durch -CO-, -O-, -S-, -CH=CH-, -C≡C-, -NH- oder -N(CH₃)- ersetzt sein können, und/ oder
Aryl, das ein oder mehrfach mit Halogen, geradkettigem, verzweigtem und/ oder cyclischem Alkyl und/ oder Aryl, das substituiert sein kann, bedeuten.

Bevorzugt sind R⁶ und R⁷ derart miteinander verbrückt, dass die Gruppe 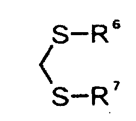 als 5- bis 7-gliedriger Ring 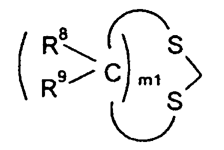 vorliegt, in der R⁸, R⁹ H oder eine gegebenenfalls substituierte Alkyl- oder Alkenylgruppe mit 1 bis 6 C-Atomen bedeutet, wobei die Gruppe 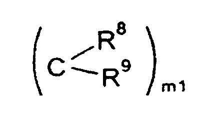 eine Cycloalkyl oder Arylgruppe ausbilden kann, und m1 2, 3 oder 4 ist.

Vorstehend und nachfolgend bedeuten die Gruppen 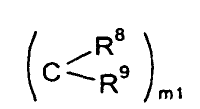 und/oder 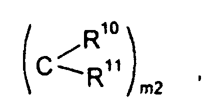 in der R¹⁰, R¹¹ und m2 eine der für R⁸, R⁹ bzw. m1 angegebenen Bedeutungen besitzen, vorzugsweise Ethylen, Propylen oder 2,2-Dimethylpropylen.

Als an das Ketendithioketal zu addierende Säure wird vorzugsweise eine Säure HY eingesetzt, wobei Y⁻ ein nicht- oder schwach-koordinierendes Anion ist. Hierbei ist Y⁻ bevorzugt ein Halogenid, Tetrafluoroborat, Hexafluorophosphat, Perchlorat oder Alkyl- oder Arylcarboxylat oder Alkyl- oder Arylsulfonat-Anion ist, wobei in den Alkyl- oder Arylgruppen ein, mehrere oder alle H-Atome durch Fluor oder Chlor substituiert sein können. Besonders bevorzugte Säuren sind Trifluormethansulfonsäure und ein Tetrafluoroborsäure-Diethyletherkomplex.

Die Säure wird in etwa äquimolarer Menge bezogen auf die umzusetzenden Ketendithioketal-Einheiten eingesetzt. Die Umsetzung mit der Säure HY erfolgt vorteilhaft in einem Temperaturbereich von -80 bis +30°C in einem inerten polaren Lösungsmittel oder Lösungsmittelgemisch. Geeignete Lösungsmittel sind beispielsweise Ether oder Halogenalkane, wie Diethylether, Tetrahydrofuran, Dichlormethan oder Trichlormethan.

Durch Addition einer Säure HY an das Ketendithioketal der Formel II wird ein Bis(alkylthio)carbeniumsalz der Formel III erhalten, 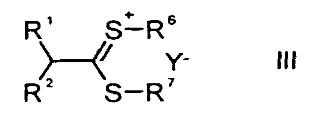 in der R¹, R², R⁶, R⁷ und Y⁻ die angegebene Bedeutung besitzen.

Von besonderem Vorteil ist, dass die Bildung des Bis(alkylthio)carbeniumsalzes durch Addition der Säure an das Ketendithioketal reversibel ist. Bei Einsatz von in 4-Stellung substituierten Cyclohexylidenketendithioketalen lassen sich daher mit sehr hoher Selektivität die gegenüber der cis-Konfiguration thermodynamisch günstigeren trans-substituierten Cyclohexanderivate des Bis(alkylthio)carbeniumsalzes und damit trans-substituierte Cycloxanverbindungen mit einer -CF₂O-Brücke erhalten.

Zur Equilibrierung zum thermodynamisch günstigeren Isomer ist es daher vorteilhaft, das Reaktionsgemisch aus Ketendithioketal, Säure und entsprechendem Carbeniumsalz eine längere Zeit, insbesondere 15 Minuten bis 6 Stunden oder auch länger, bei einer Temperatur von -80 bis +50°C, insbesondere von -30 bis +50°C, zu rühren.

Die sich an die erste Umsetzung anschließenden beiden Syntheseschritte werden vorzugsweise in situ, also unter Verwendung des Reaktionsgemisches der ersten Umsetzung und damit ohne Isolierung der Zwischenprodukte durchgeführt.

Die mindestens eine Hydroxylgruppe enthaltende Verbindung, mit der das Bis(alkylthio)carbeniumsalz umgesetzt wird, ist in ihrer Struktur an sich keinen besonderen Beschränkungen unterworfen. Da das erfindungsgemäße Verfahren jedoch besonders zur Herstellung von Flüssigkristallen selbst oder Synthesebausteinen für Flüssigkristalle geeignet ist, wird der in der mindestens eine Hydroxylgruppe enthaltenden Verbindung vorgesehene Rest vorzugsweise in der Weise ausgestaltet, dass er in Flüssigkristallen Verwendung findende Strukturelemente enthält.

Bevorzugt ist die mindestens eine Hydroxylgruppe aufweisende Verbindung ein Alkyl- oder Arylalkohol der Formel IV R³-OH (IV), in dem der Alkyl- oder Arylrest R³ beliebig substituiert sein kann.

Die Hydroxyverbindung wird vorzugsweise in einem bis zu 2-fachen, insbesondere bis zu 1,5-fachen, molaren Überschuss bezogen auf die theoretisch einzusetzende Menge verwendet. Die Umsetzung mit der Hydroxyverbindung erfolgt in Gegenwart mindestens einer Base, vorzugsweise in einem Temperaturbereich von -100 bis + 50°C. Hierzu vorteilhaft geeignete Basen sind organische Stickstoffbasen, insbesondere tertiäre Amine, wie beispielsweise Triethylamin, Pyridin oder Pyridin-Derivate. Die Base wird vorteilhaft in einem molaren Verhältnis 1 : 1 bis 2 : 1 bezogen auf die Hydroxyverbindung eingesetzt. Als geeignete Lösungsmittel kommen insbesondere polare Lösungsmittel oder Lösungsmittelgemische in Frage, wie sie bereits zuvor angeführt worden sind.

Durch Umsetzung des Bis(alkylthio)carbeniumsalzes der Formel III mit der Hydroxyverbindung der Formel IV wird ein Dithioorthoester der Formel VI 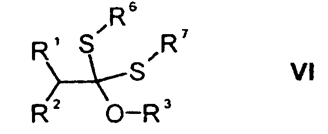 erhalten, in der R¹, R², R⁶, R⁷ und R³ die angegebene Bedeutung besitzen.

Ausgehend vom Ketendithioketal der Formel II über das durch Addition der Säure HY erhältliche Bis(alkylthio)carbeniumsalz der Formel III und den durch Umsetzung mit der Hydroxyverbindung der Formel IV zugänglichen Dithioorthoester der Formel VI ist die mindestens eine -CF₂-O-Brücke aufweisende Verbindung der Formel I erfindungsgemäß herstellbar, wie in Reaktionsschema 1 angegeben.

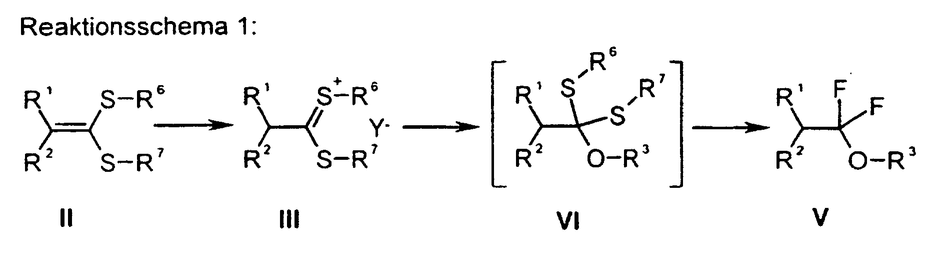

Der Orthoester VI wird im allgemeinen nicht isoliert, sondern direkt oxidativ zur Verbindung V umgesetzt.

Als Oxidationsmittel können übliche Oxidationsmittel verwendet werden. Bevorzugt wird als Oxidationsmittel eine Verbindung eingesetzt, die Haloniumäquivalente freisetzt. Beispielhafte Oxidationsmittel sind N-Chlorsuccinimid, N-Bromsuccinimid, N-Jodsuccinimid, 1,3-Dibrom-5,5-dimethylhydanthoin, Dibromisocyanursäure, Brom und Chlor. Besonders bevorzugt ist Brom, da sich die entstehenden Bromide leicht abtrennen lassen. Ebenfalls geeignet sind beispielsweise SO₂Cl₂, SO₂ClF, Nitrosonium- und Nitroniumsalze sowie Chloramin T. Die Nitrosonium- und Nitroniumsalze lassen sich gegebenenfalls auch in situ aus geeigneten Vorstufen, beispielsweise aus anorganischen oder organischen Nitriten und/ oder Nitraten, herstellen.

Als Fluorierungsmittel können übliche Fluorierungsmittel eingesetzt werden. Besonders bevorzugt wird das Fluorierungsmittel ausgewählt aus der Gruppe die gebildet ist von Fluorwasserstoff, aliphatischen und aromatischen Amin-Fluorwasserstoff-Komplexen, wie beispielsweise Pyridin-Fluorwasserstoffkomplexe, insbesondere HF in Pyridin mit einem HF-Gehalt von 50 bis 70 %, Triethylamin-, Melamin-, Polyvinylpyridin-Fluorwasserstoff-Komplexe.

Die Umsetzung mit dem Oxidations- und Fluorierungsmittel erfolgt vorteilhaft bei einer Temperatur von -100 bis +50°C. Als Lösungsmittel kommen die bereits angegebenen Lösungsmittel bzw. Lösungsmittelgemische ebenfalls in Frage.

Die gesamte Umsetzung vom Ketendithioketal bis zum Produkt mit mindestens einer -CF₂O-Brücke erfolgt besonders bevorzugt als sogenanntes Eintopfverfahren, d. h. ohne Isolierung und Aufreinigung des Carbeniumsalzes und/ oder des Dithioorthoesters als Zwischenprodukte.

Bevorzugt weist die mindestens eine Hydroxylgruppe aufweisende Verbindung die Formel IVa auf, 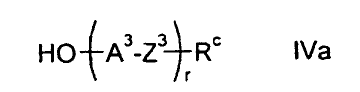 in der
- R^{c}: H, Halogen, -CN, -NCS, -SF₅ oder Alkyl mit 1 bis 18 C-Atomen, worin auch eine oder zwei nicht benachbarte -CH₂-Gruppen durch -O-, -S-, -CO-, -O-CO-, -CO-O-, -E- und/ oder -C=C- ersetzt sein können und/ oder worin auch eine oder mehrere H-Atome durch Halogen und/ oder -CN ersetzt sein können, bedeutet,
- E: CR⁴=CR⁵ oder CHR⁴-CHR⁵,
- R⁴, R⁵: jeweils unabhängig voneinander H, Alkyl mit 1-6 C-Atomen, F, Cl, Br, CF₃ oder CN bedeutet und
- Z³: -O-CO-, -CO-O-, -C₂H₄-, -CH₂-CF₂-, -CF₂-CH₂-, -CF₂-CF₂-, -CH₂₋O-, -CF₂-O-, -O-CH₂-, -O-CF₂-, -CH=CH-, -CF=CH-, -CH=CF-, -CF=CF-, -C≡C- oder eine Einfachbindung, und
- A³: 1,4-Phenylen, worin ein oder mehrere CH-Gruppen durch N ersetzt sein können, 1,4-Cyclohexylen, worin ein oder zwei nicht benachbarte CH₂-Gruppen durch O und/ oder S ersetzt sein können, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei in diesen Gruppen ein oder mehrere H-Atome substituiert sein können durch Halogen, -CN und/ oder Alkyl mit 1 bis 6 C-Atomen, worin ein oder mehrere H-Atome durch Halogen oder -CN, und/ oder worin ein oder mehrere nicht benachbarte -CH₂-Gruppen unabhängig voneinander durch -CO-, -O-CO-, -CO-O-, -O-, -S-, -CH=CH-, -C≡C-, -NH- oder -N(CH₃)- ersetzt sein können, und
- r: 0, 1 oder 2 ist,
mit der Maßgabe, dass im Falle r = 0 R^{c} die angegebene Bedeutung Alkyl hat, bei der das C-Atom in 1-Position jedoch nicht durch ein Heteroatom ersetzt ist.

Bevorzugt ist A³ 1,4-Phenylen, das in 2, 3, 4 und/ oder 5-Position wie angegeben, vorzugsweise durch Fluor, substituiert sein kann.

Im Fall r = 0 ist die Hydroxyverbindung bevorzugt ein Alkanol, das substituiert sein kann. Besonders bevorzugt sind halogenierte Alkanole, beispielsweise Trifluormethanol, Trifluorethanol und Trichlorethanol.

Gemäß einer weiteren bevorzugten Variante des erfindungsgemäßen Verfahrens weist die mindestens eine Hydroxylgruppe aufweisende Verbindung zwei Hydroxylgruppen auf.
Durch die erfindungsgemäße Umsetzung mit zwei gleichen oder zwei unterschiedlichen Bis(alkylthio)carbeniumsalzen können mit solchen Dihydroxyverbindungen Verbindungen erhalten werden, die zwei -CF₂-O-Brücken aufweisen.

Gemäß dieser Variante besitzt die Dihydroxyverbindung bevorzugt die Formel IVd 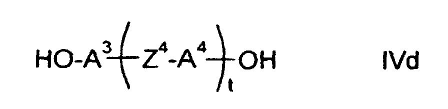 in der A³ die angegebene Bedeutung aufweist und A⁴ und Z⁴ eine der für A³ bzw. Z³ angegebenen Bedeutungen besitzen und t 0, 1 oder 2 ist.

Ganz besonders bevorzugt wird das Ketendithioketal aus einer Carbonylverbindung erhalten. Die Carbonylverbindung kann mit der Formel I beschrieben werden, 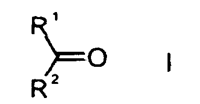 in der R¹ und R² die angegebenen Bedeutungen besitzen.

Die Ketendithioketale sind aus den Carbonylverbindungen nach an sich bekannten Verfahren auf einfache Weise und in hohen Ausbeuten zugänglich. Beispielhaft sei hier D. J. Ager, Org. React. 1990, 38, 1 - 223, insbesondere die Seiten 63, 95 und 96 genannt. Von Vorteil ist, dass die Carbonylverbindungen zusätzlich säurelabile Substituenten enthalten können. Wie bereits eingangs genannt, ist ein weiterer Vorteil, dass die hieraus erhältlichen Ketenthioketale in der Regel durch ihre guten Kristallisationseigenschaften gut zu reinigen sind, wobei dennoch eine gute Löslichkeit in üblichen organischen Lösungsmitteln gegeben ist.

Ein hierbei bevorzugtes Verfahren ist die Umsetzung einer Carbonylverbindung mit einem 2-Silyl-1,3-dithian, das substituiert sein kann. Besonders bevorzugt ist hierbei der Einsatz von 2-Trimethylsilyl-1,3-dithian. Die Umsetzung erfolgt vorzugsweise in Gegenwart einer deprotonierenden Verbindung, wie Alkyllithium, beispielsweise n-Butyllithium. Ein vorteilhafter Bereich der Reaktionstemperatur ist -130 bis 0°C. Geeignete Lösungsmittel sind die zuvor angegebenen Lösungsmittel bzw. Gemische.

Hierbei bevorzugte Carbonylverbindungen sind solche gemäß der Formel Ia, die sich aufgrund der vorhandenen Strukturelemente vorteilhaft zur Herstellung von flüssigkristallinen Verbindungen oder Bausteinen für die Flüssigkristallsynthese eignen,

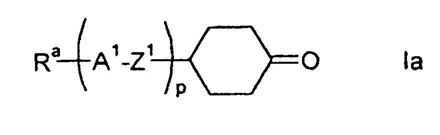

Hierin bedeuten
- R^{a}: H, Halogen, -CN oder Alkyl mit 1 bis 18 C-Atomen, worin auch eine oder zwei nicht benachbarte -CH₂-Gruppen durch -O-, -S-, -CO-, -O-CO-, -CO-O-, -E- und/ oder -C≡C- ersetzt sein können und/ oder worin auch eine oder mehrere H-Atome durch Halogen und/ oder CN ersetzt sein können,
- E: CR⁴=CR⁵ oder CHR⁴-CHR⁵,
- R⁴, R⁵: jeweils unabhängig voneinander H, Alkyl mit 1-6 C-Atomen, F, Cl, Br, CF₃ oder CN bedeutet und
- Z¹: -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- oder eine Einfachbindung, und
- A¹: 1,4-Phenylen, worin ein oder mehrere CH-Gruppen durch N ersetzt sein können, 1,4-Cyclohexylen, worin ein oder zwei nicht benachbarte CH₂-Gruppen durch O und/ oder S ersetzt sein können, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei in diesen Gruppen ein oder mehrere H-Atome substituiert sein können durch Halogen, -CN und/ oder Alkyl mit 1 bis 6 C-Atomen, worin ein oder mehrere H-Atome durch Halogen oder -CN, und/ oder worin ein oder mehrere nicht benachbarte -CH₂-Gruppen unabhängig voneinander durch -CO-, -O-CO-, -CO-O-, -O-, -S-, -CH=CH-, -C≡C-, -NH- oder -N(CH₃)- ersetzt sein können, und
- p: hat den Wert 0, 1 oder 2.

Im Falle der Bedeutung Alkyl in den vorstehend oder nachfolgend angegebenen Gruppen oder Substituenten, insbesondere in R^{a}, R^{b}, R^{c} und/ oder R^{d}, kann der Alkyl-Rest linear oder verzweigt sein. Bevorzugt besitzt er 1, 2, 3, 4, 5, 6, 7 oder 8 C-Atome. Bevorzugt ist er linear und bedeutet daher besonders Methyl, Ethyl, Propyl, Butyl, Pentyl, Hexyl, Heptyl oder Octyl. Ein verzweigter Alkylrest kann chiral oder achiral sein. Bevorzugte chirale Alkylreste sind 2-Butyl (=1-Methylpropyl), 2-Methylbutyl, 2-Methylpentyl, 3-Methylpentyl, 2-Ethylhexyl, 2-Propylpentyl, 2-Octyl. Bevorzugte achirale Alkylreste sind Isopropyl, Isobutyl (=Methylpropyl), Isopentyl (=3-Methylbutyl). Die Alkylreste können in der angegebenen Weise substituiert sein.

Unter Einsatz von Carbonylverbindungen der Formel Ia und Hydroxyverbindungen der Formel IVa sind nach dem erfindungsgemäßen Verfahren die -CF₂-O-verbrückten Verbindungen der Formel Va leicht zugänglich. Dieser Syntheseweg wird anhand des Reaktionsschemas 2 beschrieben, in dem die Formeln IIa die Ketendithioketale und IIIa die entsprechenden Carbeniumsalze bezeichnen.

In dem Reaktionsschema 2 und den folgenden Reaktionsschemata ist jeweils die Zwischenstufe des Dithioorthoesters der Übersichtlichkeit halber nicht explizit dargestellt.

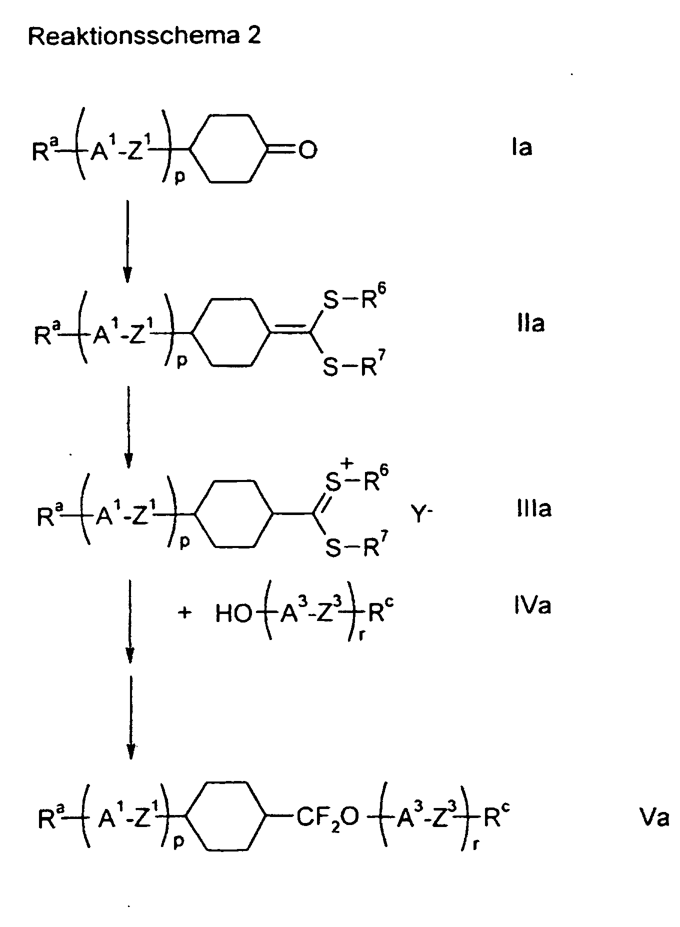

Wird anstatt der Hydroxyverbindung der Formel IVa die zuvor beschriebene Dihydroxyverbindung der Formel IVd eingesetzt, so lassen sich hieran zwei gleiche oder unterschiedliche Bis(alkylthio)carbeniumsalze der Formeln IIIa und IIId addieren, wobei nach der oxidativen Fluorodesulfurierung gemäß Reaktionsschema 3 Verbindungen der Formel Vd mit zwei CF₂-O-Brücken erhalten werden. Diese Carbeniumsalze sind über die entsprechenden Ketendithioketale der Formeln IIa und IId aus den Carbonylverbindungen Ia und Id zugänglich.

Nachfolgend haben R¹⁰, R¹¹, m2, Z², A², q, R^{b}, Y'⁻ jeweils eine der für R⁸, R⁹, m1, Z¹, A¹, p, R^{a}, Y⁻ angegebenen Bedeutungen.

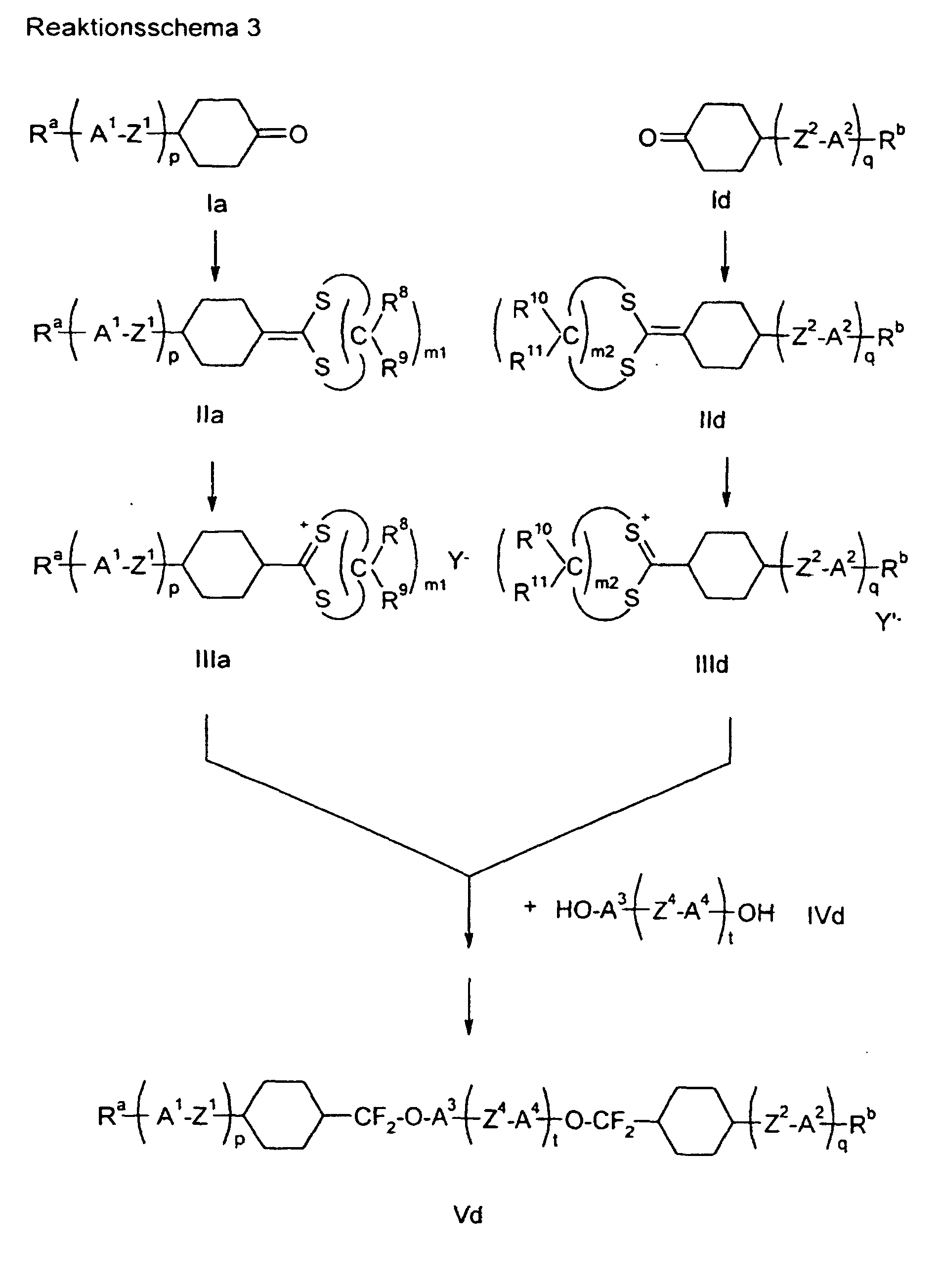

Weitere bevorzugte Carbonylverbindungen sind solche mit zwei oder mehr Carbonylgruppen.

Hierbei zwei besonders bevorzugte Dicarbonylverbindungen sind Cyclohexandion der Formel Ib 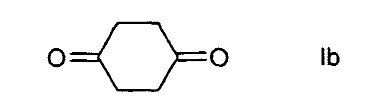 und die Biscyclohexanon-Verbindung der Formel Ic 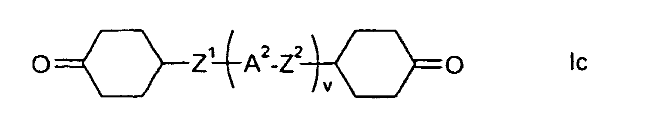 in der Z¹, Z² jeweils unabhängig voneinander einer der für Z¹ angegebenen Bedeutungen und A² eine der für A¹ angebenen Bedeutungen besitzen und v den Wert 0, 1 oder 2 besitzt.

Mit solchen zwei oder mehr Carbonylfunktionen aufweisenden Verbindungen können nach einer ersten Variante des erfindungsgemäßen Verfahrens Verbindungen mit zwei oder mehreren -CF₂-O-Brücken im Molekül erhalten werden. Die entsprechenden Zwischenstufen weisen je Molekül zwei oder mehr Ketendithioketal-Funktionen und zwei oder mehr Carbenium-Funktionen auf.

Gemäß einer zweiten Variante des erfindunsgemäßen Verfahrens werden eine oder mehrere Carbonylgruppen solch einer Carbonylverbindung vor der Umsetzung zum entsprechenden Ketendithioketal als Ketal geschützt, wobei mindestens eine Carbonylgruppe zur Umsetzung zum Ketendithioketal ungeschützt bleibt. Die hieraus zugänglichen Verbindungen weisen neben einer oder mehreren -CF₂-O-Brücken zusätzlich eine oder mehrere Carbonylfunktionen, gegebenenfalls geschützt als Ketal, auf. Insbesondere bei der Flüssigkristall-Synthese kann die freie Carbonyl-Funktion vorteilhaft zum Aufbau einer gegebenenfalls substituierten Alkyl-, Alkenyl- oder Alkoxygruppe verwendet werden.

Hierbei ist das Ketal vorzugsweise die Gruppe 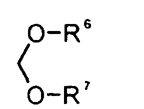 mit den angegebenen Bedeutungen für R⁶ und R⁷.
Bevorzugt sind R⁶ und R⁷ derart miteinander verbrückt, dass die Gruppe 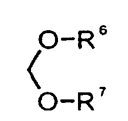 als 5- bis 7-gliedriger Ring 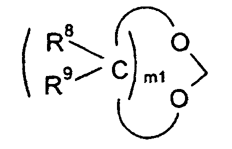 vorliegt, in der R⁸, R⁹ und m1 die angegebenen Bedeutungen besitzen.

Das Reaktionsschema 4 veranschaulicht ausgehend vom Cyclohexandion der Formel Ib die erste Variante des Verfahrens, gemäß der über die Zwischenstufe IIb2 mit zwei Ketendithioketal-Funktionen und IIIb2 mit zwei Carbenium-Funktionen durch Addition von zwei gleichen oder unterschiedlichen Hydroxyverbindungen der Formeln IVa und IVb und anschließender oxidativer Fluorodesulfurierung Verbindungen der Formel Vb2 mit zwei -CF₂-O-Brücken erhalten werden.

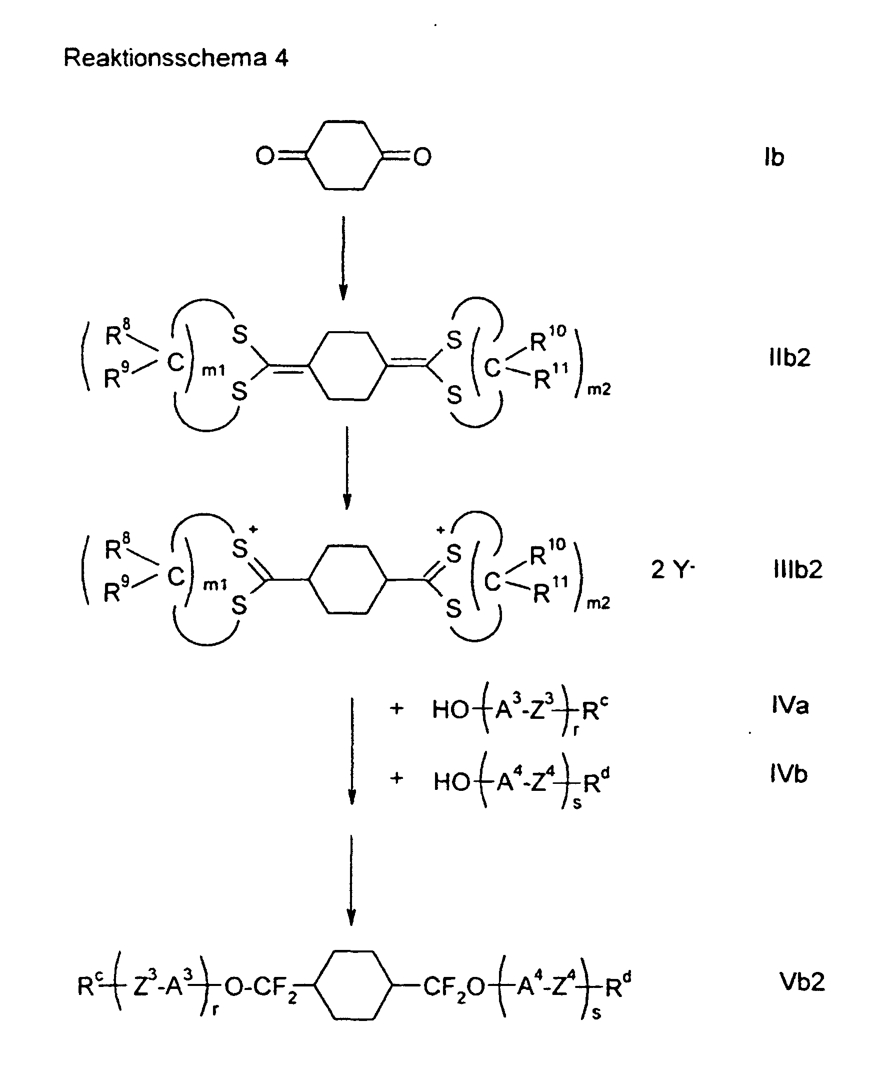

In analoger Weise werden ausgehend von Biscyclohexandionen der Formel Ic über die Bisketendithioketale IIc2 und die Biscarbeniumsalze IIIc2 durch Addition der beiden gleichen oder unterschiedlichen Hydroxyverbindungen IVa und IVb Verbindungen der Formel Vc2 mit zwei -CF₂-O-Brücken erhalten (Reaktionsschema 5).

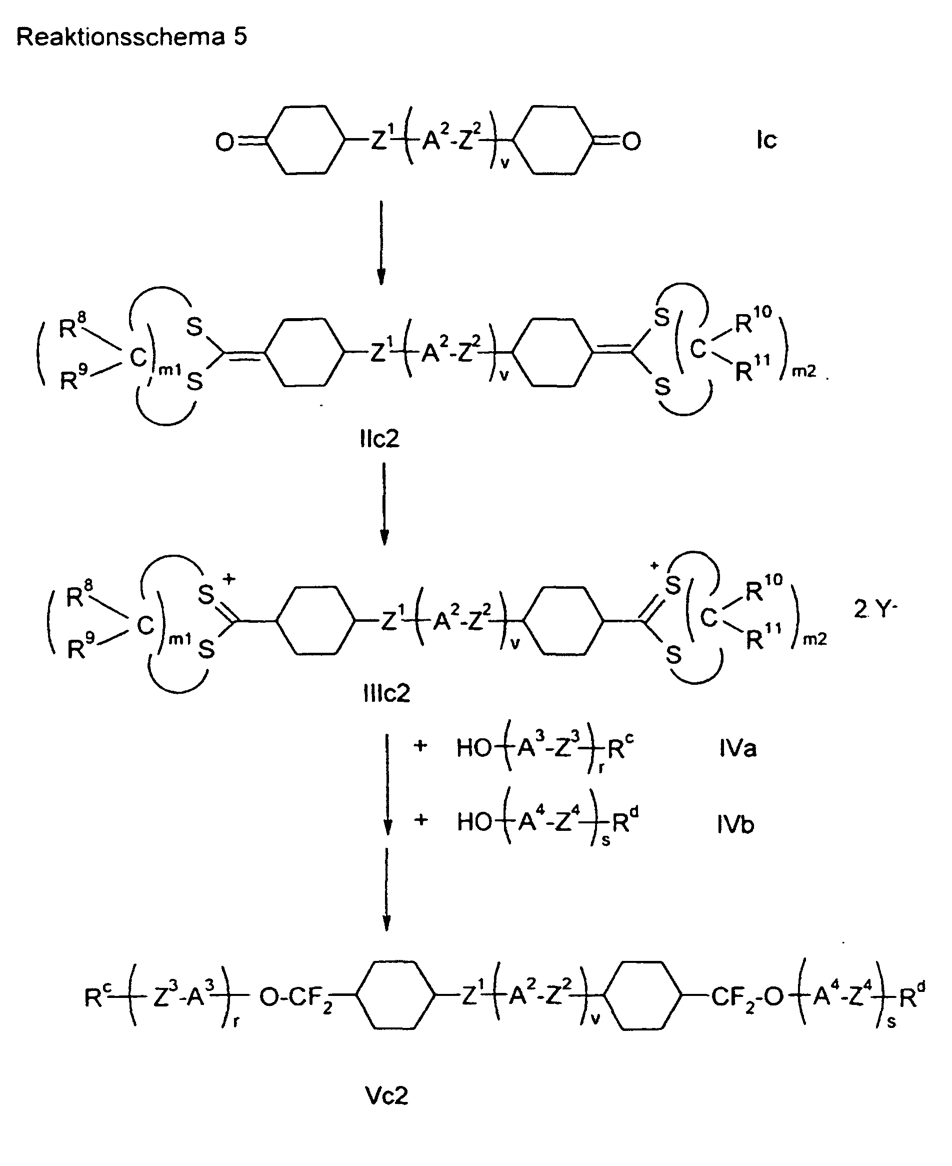

Die zweite Variante des Verfahrens unter Einsatz von Cyclohexandion wird anhand des Reaktionsschemas 6 veranschaulicht. Im ersten Schritt wird nach dem Fachmann bekannten Verfahren eine Carbonylfunktion des Cyclohexandions der Formel Ib als Ketal geschützt. Die so erhaltene eine freie Carbonylgruppe aufweisende Verbindung der Formel Ib1 wird erfindungsgemäß zum Ketendithioketal IIb1 und weiter zum Bis(alkylthio)-carbeniumsalz IIIb1 umgesetzt, an das eine Hydroxyverbindung der Formel IVa addiert wird. Die nach oxidativer Fluorodesulfurierung erhaltene Verbindung der Formel Vb1 weist eine -CF₂-O-Brücke und eine als Ketal geschützte Carbonylgruppe auf. Die Verbindung Vb1 kann unmittelbar weiter als Synthesebaustein dienen, beispielsweise in der Flüssigkristallsynthese, oder wie hier dargestellt, das Ketal wird nach dem Fachmann bekannten Verfahren gespalten unter Erhalt der freien Carbonylfunktion in der Verbindung der Formel Vb.

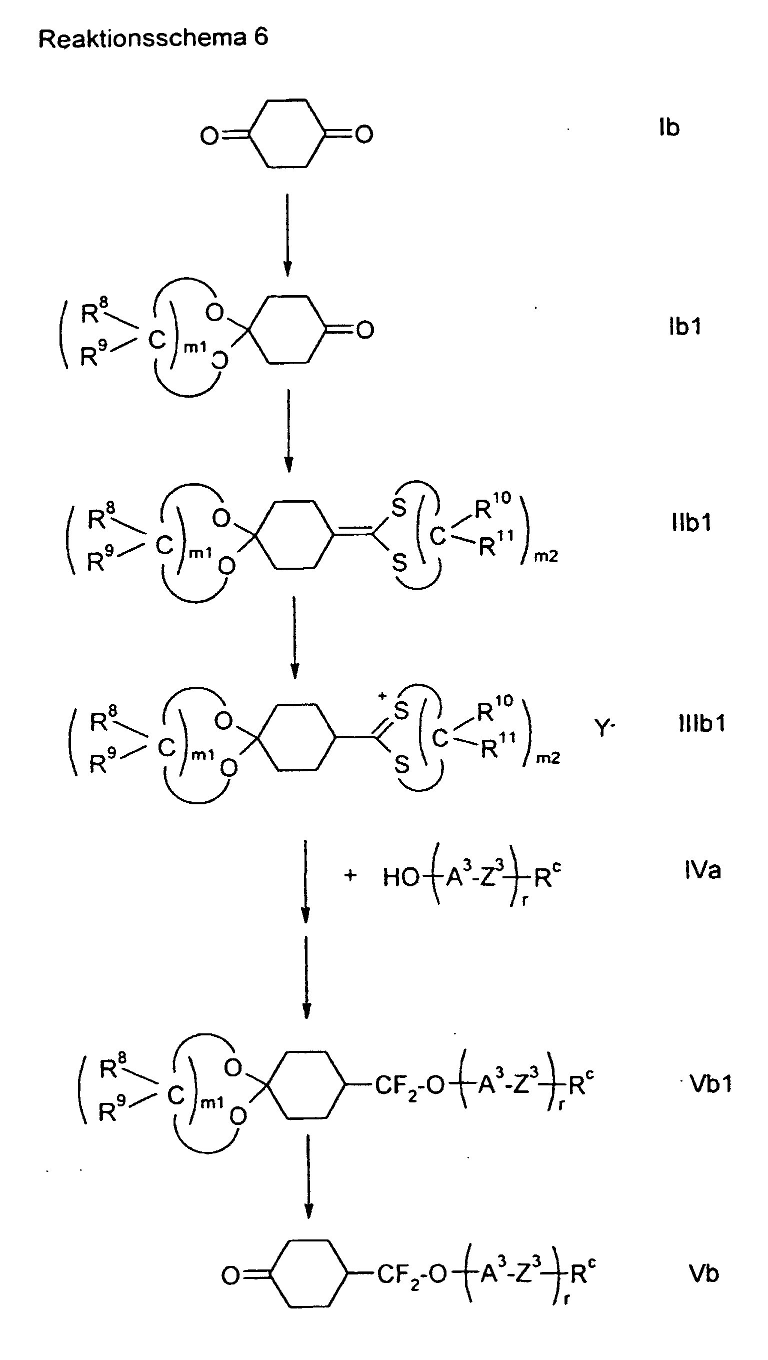

In analoger Weise ist die zweite Variante des Verfahrens mit Biscyclohexanonen der Formel Ic gemäß Reaktionsschema 7 durchführbar, wobei im ersten Schritt eine Carbonylgruppe als Ketal geschützt wird unter Erhalt der Verbindung Ic1. Die freie Carbonylgruppe wird zum entsprechenden Ketendithioketal (IIc1) und weiter zum entsprechenden Carbeniumsalz (IIIc1) umgesetzt, an das die Hydroxyverbindung IVa addiert wird. Die so erhaltene Verbindung Vc1 kann durch Spaltung des Ketals in die Cyclohexanon-Verbindung der Formel Vc umgewandelt werden.

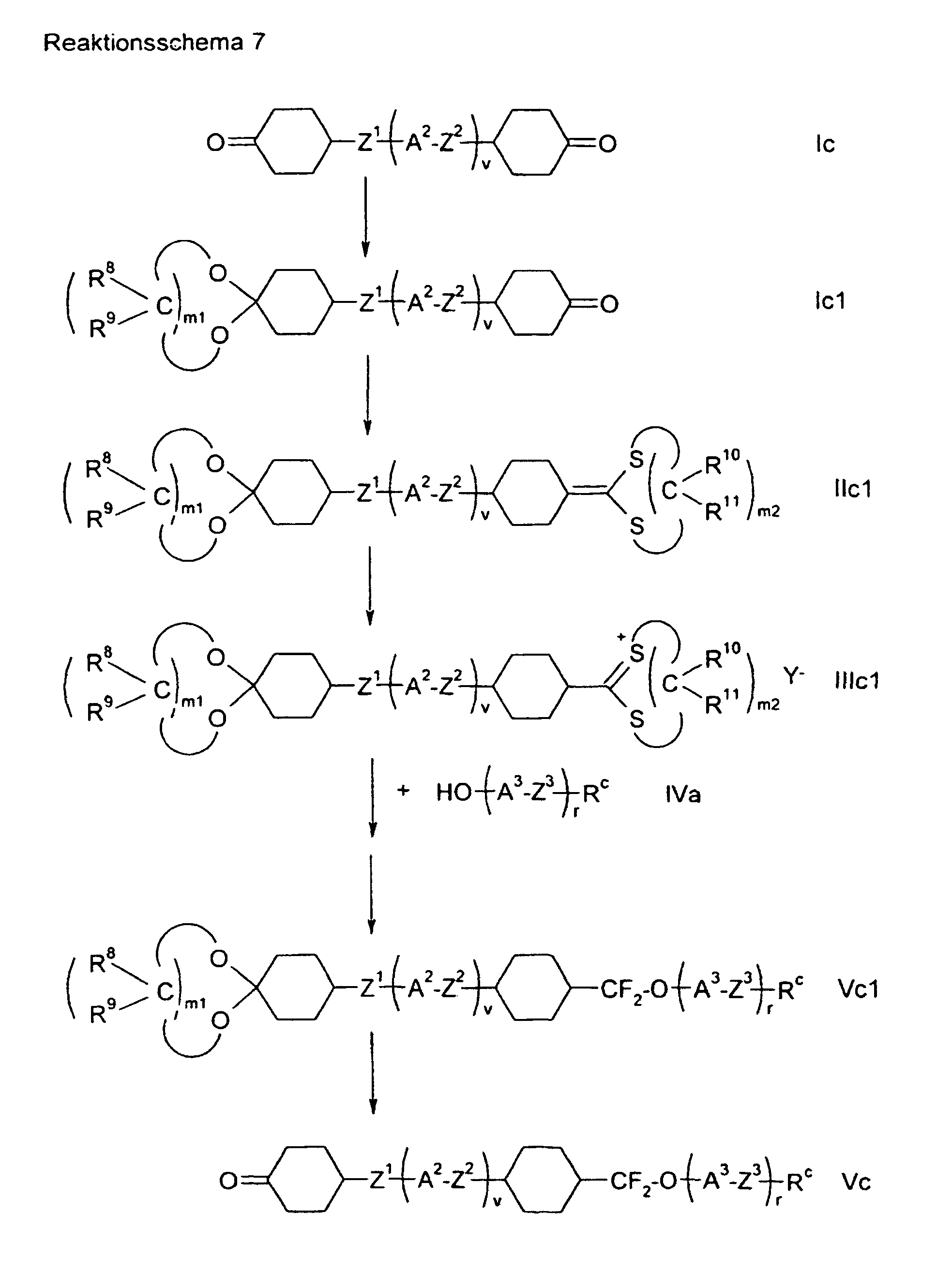

Das erfindungsgemäße Verfahren eignet sich allgemein zur Herstellung von Verbindungen mit einer, zwei oder mehreren -CF₂-O-Brücken wie z.B. Flüssigkristalle, Vorstufen für Polymere, Pharmazeutika und Pflanzenschutzmittel. Besonders geeignet ist es jedoch zur Herstellung flüssigkristalliner Verbindungen.

Bevorzugte flüssigkristalline Verbindungen, die nach dem erfindungsgemäßen Verfahren vorteilhaft zugänglich sind, sind nachfolgend aufgeführt, wobei R^{a}, R^{b}, R^{c}, Z¹, Z² und p die angegebenen Bedeutungen besitzen.

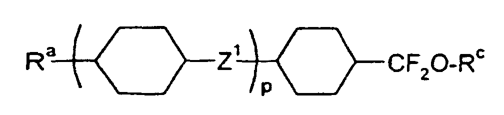

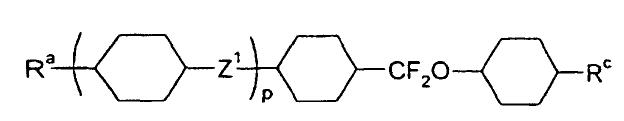

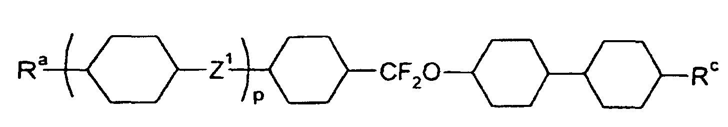

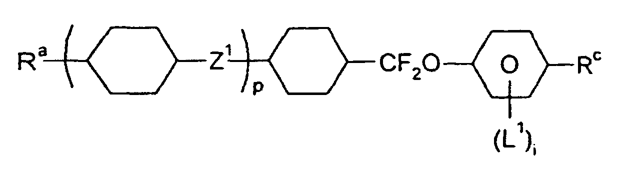

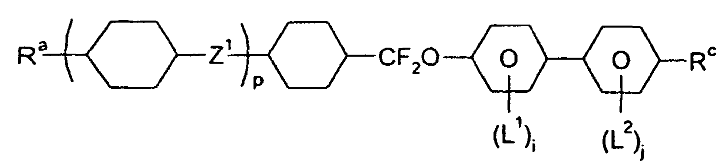

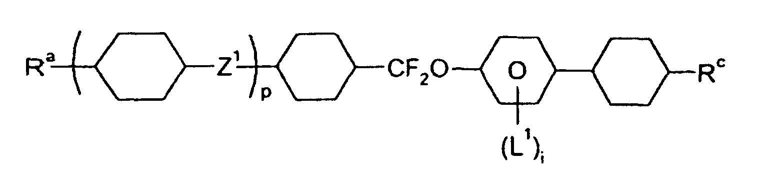

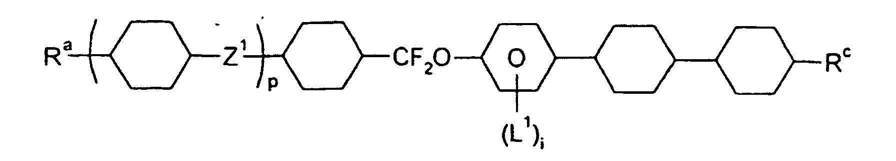

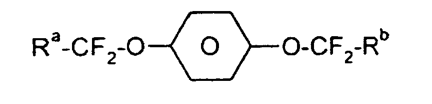

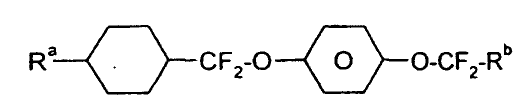

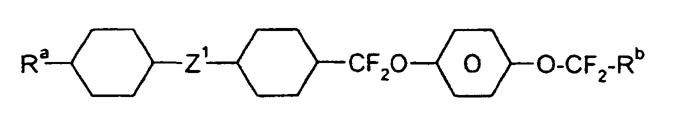

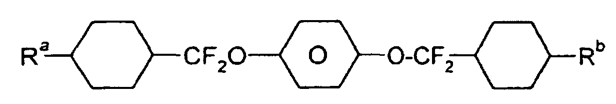

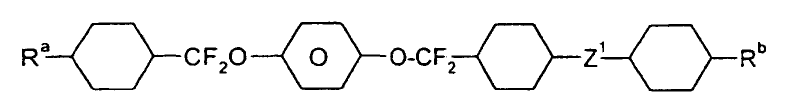

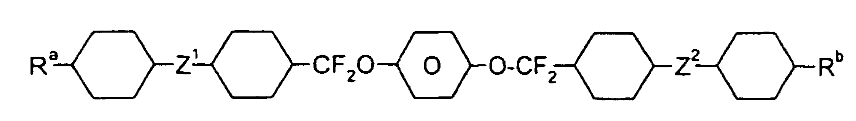

Vorstehend und nachfolgend bedeuten L¹, L² unabhängig voneinander F, Cl, eine Alkyl-, Alkenyl-, Alkoxy- und/ oder Alkenyloxy-Gruppe mit 1 bis 6 C-Atomen, in der ein oder mehrere H-Atome durch Fluor substituiert sein können, und i, j unabhängig voneinander 0, 1, 2, 3 oder 4, vorzugsweise 0, 1 oder 2.

Die Gruppen 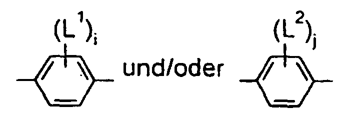 bedeuten vorzugsweise 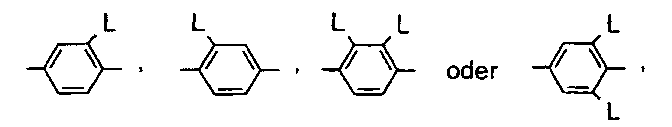 wobei L eine der für L¹, L² angegebenen Bedeutungen, insbesondere F, aufweist.

Nachfolgend werden die bevorzugten neuen Edukte und Zwischenprodukte des erfindungsgemäßen Verfahrens sowie die bevorzugten neuen, nach dem erfindungsgemäßen Verfahren erhältlichen Produkte, die zusammen einen Gegenstand der Erfindung darstellen, näher erläutert.

Neben den vorstehend und nachfolgend dargestellten neuen Ketendithioketalen, Bis(alkylthio)carbeniumsalzen und Verbindungen mit mindestens einer -CF₂O-Brücke sind erfindungsgemäß auch die entsprechenden, hieraus ableitbaren Dithioorthoester umfasst.

Vor- und nachstehend sind von den Verbindungen mit mindestens einer 1,4-substituierten Cyclohexylen-Gruppe diejenigen bevorzugt, in der diese Gruppe trans-ständig substituiert ist.

Bevorzugte Ketendithioketale und die entsprechenden hiervon abgeleiteten Carbeniumsalze, die sich vorteilhaft als Edukte für die Flüssigkristallsynthese eignen, sind nachfolgend aufgeführt:

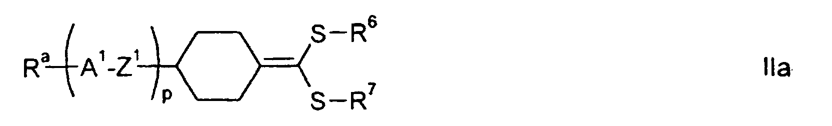

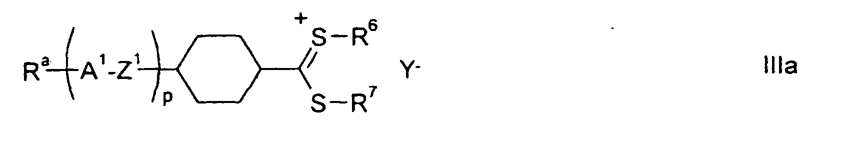

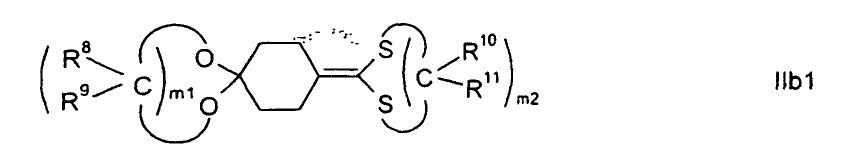

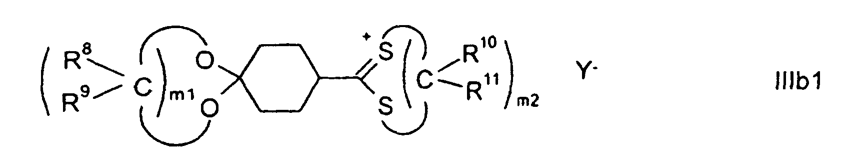

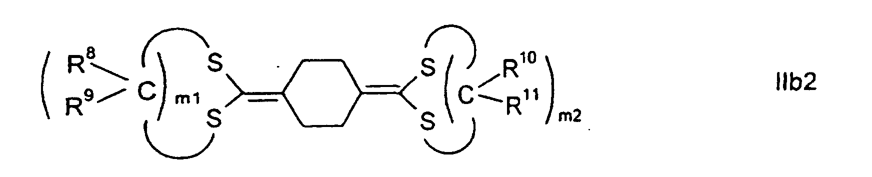

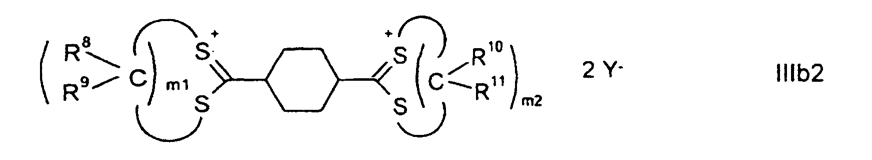

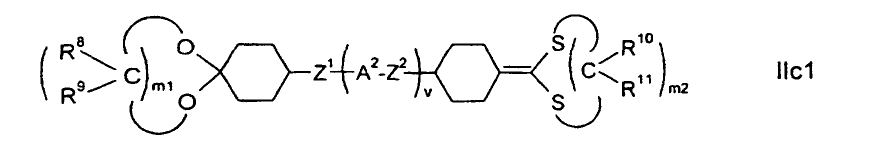

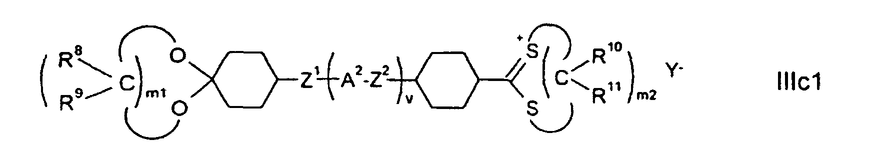

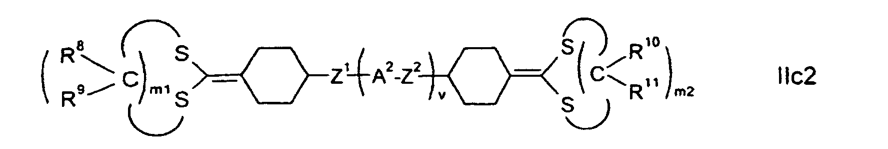

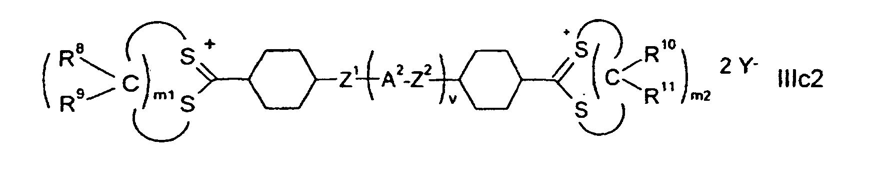

Vor- und nachstehend haben die in den Formeln für die erfindungsgemäßen Verbindungen verwendeten Substituenten, Gruppen und Indizes jeweils eine der bereits zu dem erfindungsgemäßen Verfahren angegebenen Bedeutungen.

Neu sind insbesondere die nach dem erfindungsgemäßen Verfahren erhältlichen Produkte, die sowohl mindestens eine -CF₂O-Brücke als auch mindestens eine Carbonyl-Gruppe, die als Ketal geschützt sein kann, aufweisen. Hierbei bevorzugt sind diejenigen Verbindungen, die eine Cyclohexanon-Gruppe aufweisen. Nachfolgend werden hiervon die besonders bevorzugten Verbindungen angegeben:

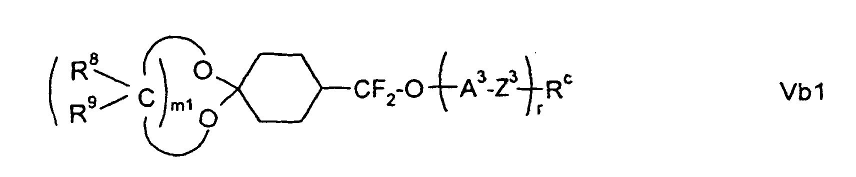

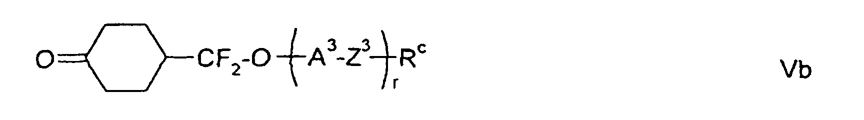

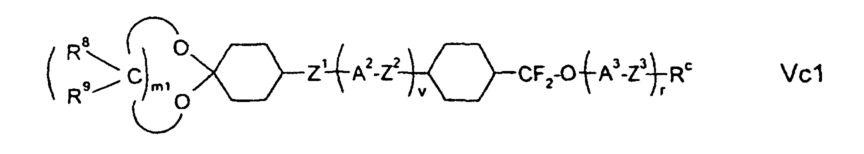

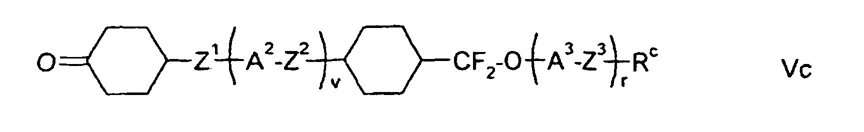

Von den Verbindungen der Formeln Vb1 und Vb sind die nachfolgenden Carbonyl-Verbindungen bevorzugt:

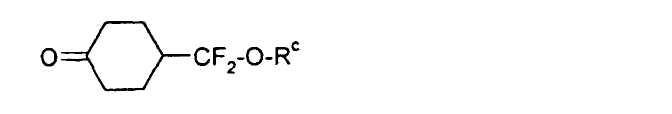

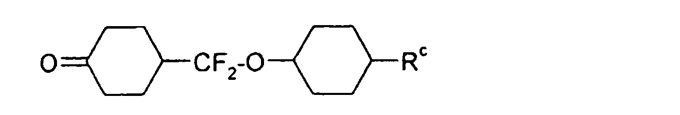

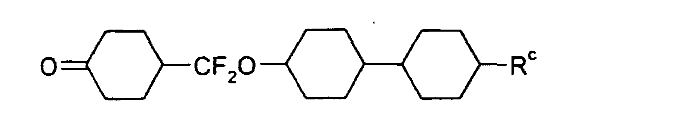

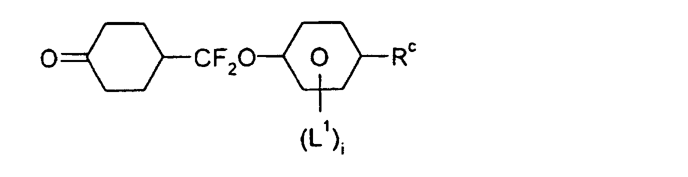

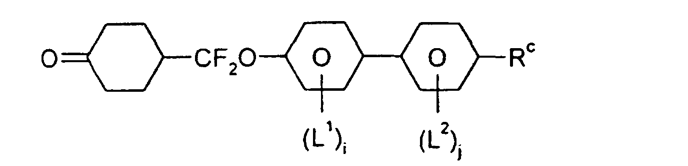

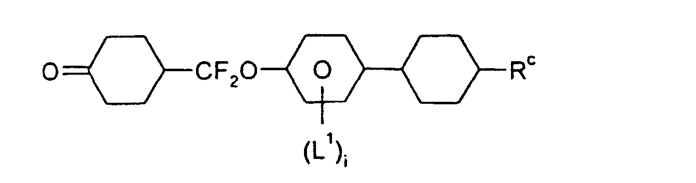

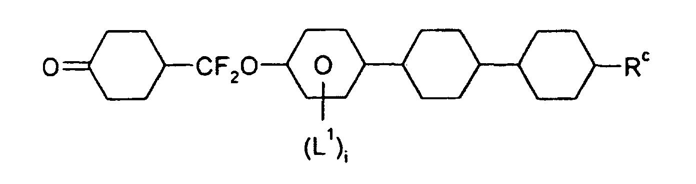

Die entsprechenden Verbindungen, bei denen die Carbonylfunktion als Ketal geschützt ist, sind ebenfalls als bevorzugt umfasst, jedoch der Übersichtlichkeit halber nicht explizit dargestellt.

Des weiteren sind von den Verbindungen der Formeln Vc1 und Vc die nachfolgenden Carbonyl-Verbindungen bevorzugt, wobei die entsprechenden, die als Ketal geschützte Carbonylfunktion aufweisenden Verbindungen ebenfalls bevorzugt sind:

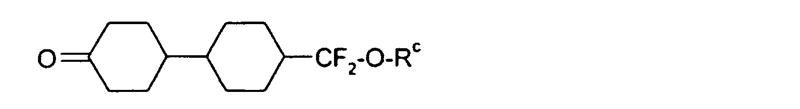

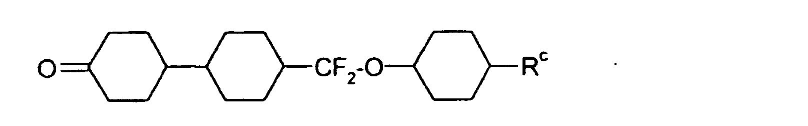

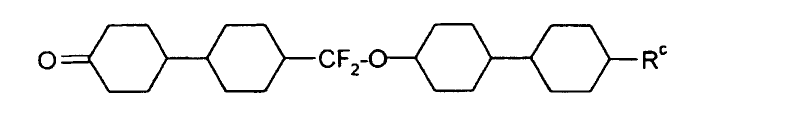

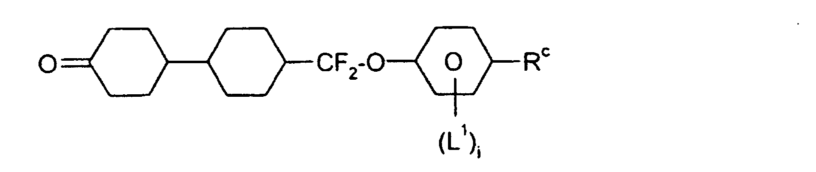

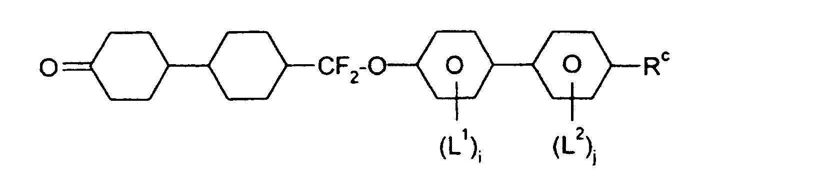

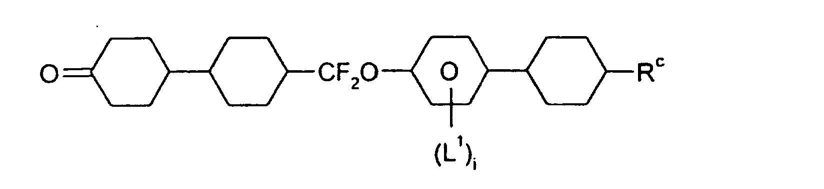

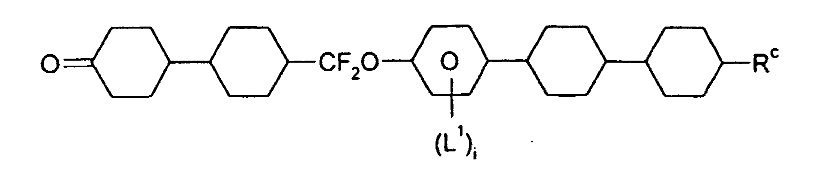

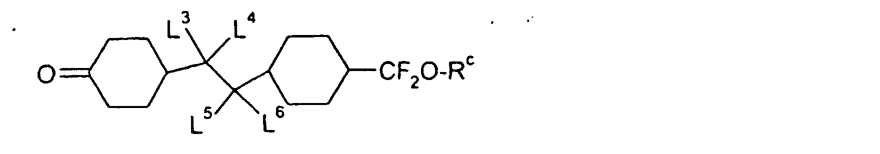

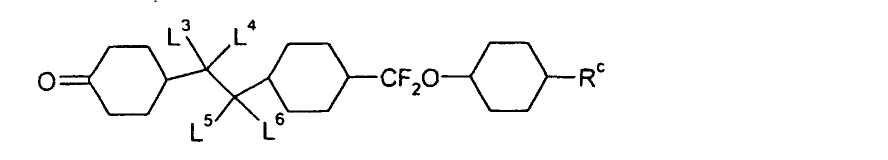

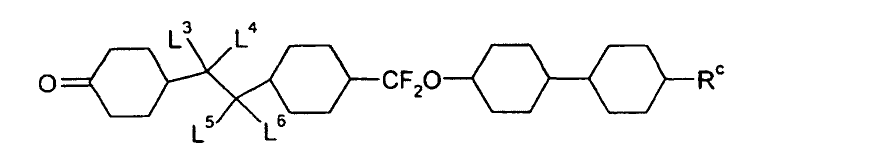

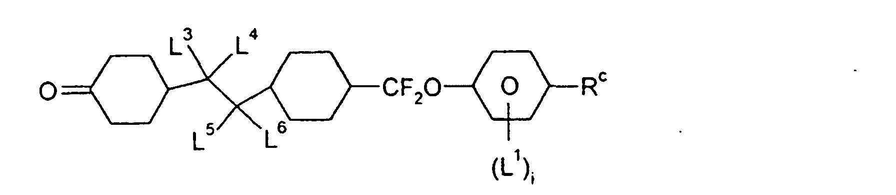

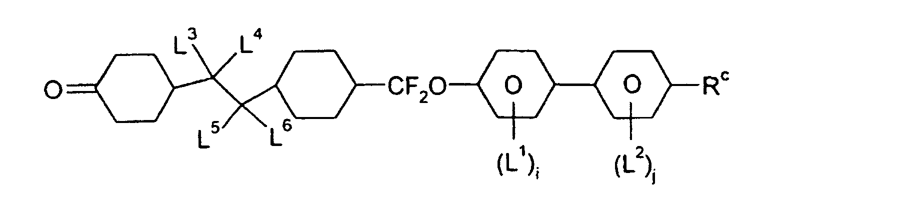

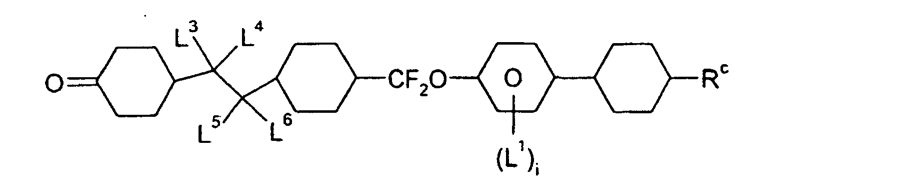

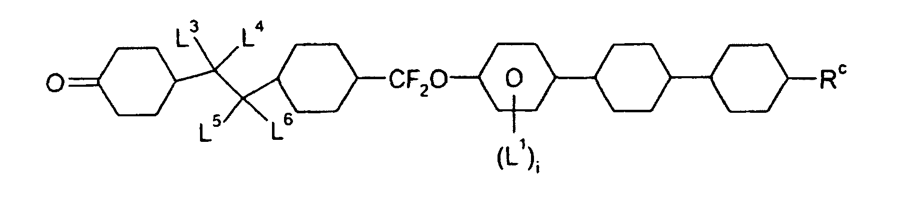

In den vorstehenden Formeln weisen L³, L⁴, L⁵ und L⁶ unabhängig voneinander eine der zuvor für L¹, L² angegebenen Bedeutungen, vorzugsweise H oder F, auf. Besonders bevorzugte Bedeutungen der Gruppe 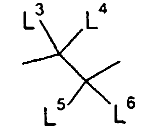 sind -CH₂-CH₂-, -CF₂-CH₂-, -CH₂-CF₂- oder -CF₂-CF₂-.

Neu sind ebenfalls das Diketon der Formel Ic.1 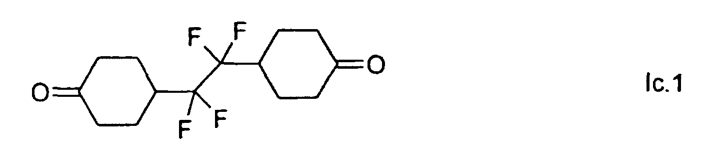 und die entsprechenden Ketale der Formel Ic1.1 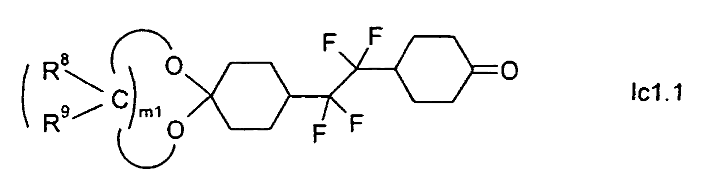 in denen R⁸, R⁹ und m1 die zuvor angegebenen Bedeutungen besitzen, als Ausgangsverbindungen zur erfindungsgemäßen Synthese, insbesondere der zuvor aufgeführten Verbindungen mit einer 1,2-Biscyclohexyl-tetrafluorethylen-Gruppe.

Besonders bevorzugte Ketale der Formel Ic1.1 sind solche der Formeln Ic1.1a bis Ic1.1c.

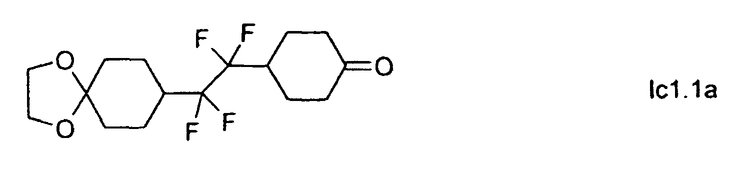

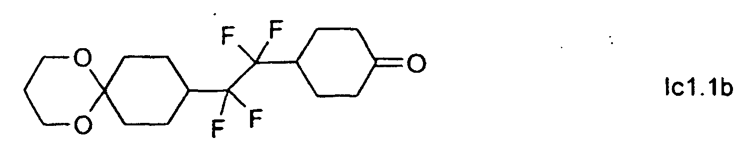

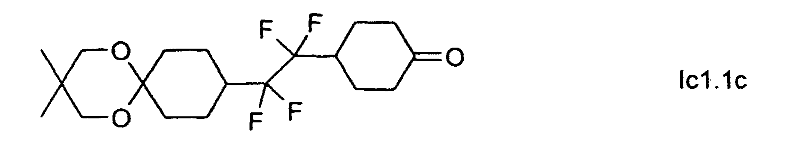

Die folgenden Ausführungsbeispiele sollen die Erfindung erläutern, ohne sie zu begrenzen. Vor- und nachstehend bedeuten Prozentangaben Gewichtsprozent. Alle Temperaturen sind in Grad Celsius angegeben. Es bedeuten K = kristalliner Zustand, N = nematische Phase und I = isotrope Phase. Die Angaben zwischen diesen Symbolen stellen die Übergangstemperaturen dar. Δε bedeutet dielektrische Anisotropie (1 kHz, 20 °C) und Δn optische Anisotropie (589 nm, 20 °C).

"Übliche Aufarbeitung" bedeutet: man gibt gegebenenfalls Wasser hinzu, stellt, falls erforderlich, je nach Konstitution des Endprodukts auf pH-Werte zwischen 1 und 11 ein, extrahiert mit einem geeigneten Lösungsmittel, beispielsweise Dichlormethan, Diethylether, Methyl-tert.-butylether oder Toluol, trennt ab, trocknet die organische Phase, dampft ein und reinigt das Produkt durch Destillation unter reduziertem Druck oder Kristallisation und/ oder Chromatographie.

Es werden die Abkürzungen THF für Tetrahydrofuran, MTB für Methyl-tert.-butylether und DBH für 1,3-Dibrom-5,5-dimethythydanthoin verwendet.

### Ausführungsbeispiele

### 1. Synthese der flüssigkristallinen Verbindung der Formel V.1

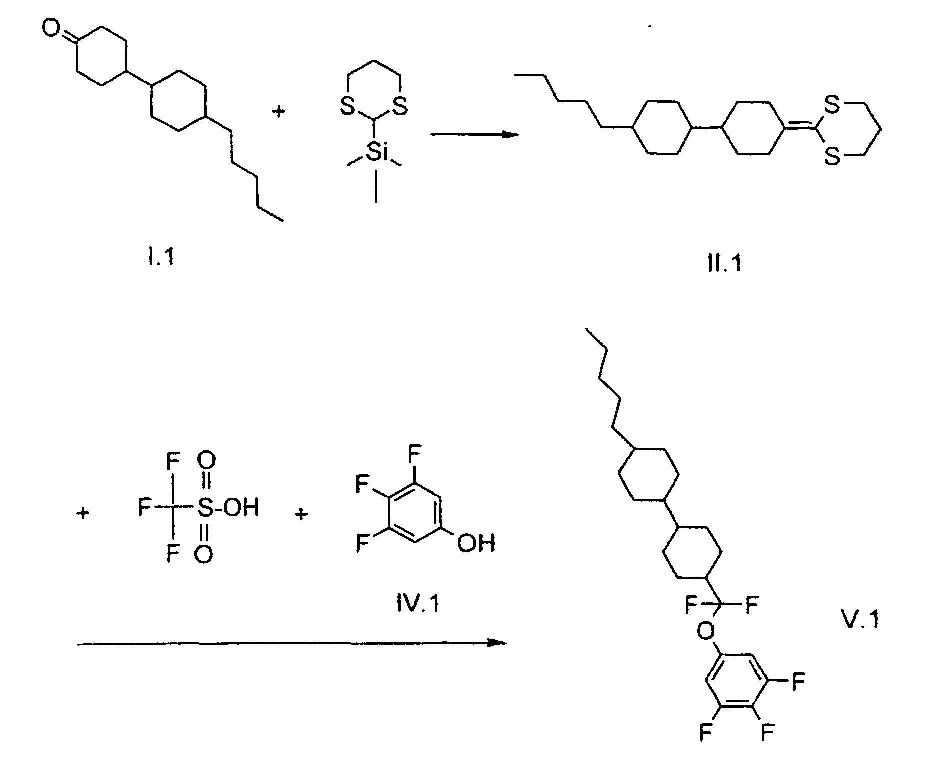

### 1.1 Herstellung der Verbindung II.1

0,075 mol 2-Trimethylsilyl-1,3-dithian wurden in 150 ml THF gelöst und bei -70 °C Butyllithium (0,078 mol als 15%ige Lösung in n-Hexan) zugetropft. Man ließ innerhalb von 4 h allmählich auf 0 °C erwärmen, kühlte erneut auf -70 °C und ließ das Keton in 50 ml THF zutropfen. Weitere 100 ml THF wurden hinzugefügt. Nach beendeter Zugabe wurde die Kühlung entfernt und über Nacht gerührt. Anschließend hydrolysierte man mit 50 ml gesättigter Natriumhydrogencarbonatlösung, fügte 500 ml Petrolether hinzu, wusch dreimal mit je 100 ml Wasser und trocknete über Natriumsulfat. Das Lösungsmittel wurde im Vakuum entfernt und das leicht gelbe Rohprodukt aus n-Hexan umkristallisiert. Man erhielt farblose Nadeln.

### 1.2 Herstellung der Verbindung V.1

Das Ketendithioketal II.1 (2,84 mmol) wurde in 15 ml Dichlormethan gelöst und unter Eiskühlung 2,84 mmol Trifluormethansulfonsäure zugetropft. Nach 15 min wurde die Kühlung entfernt und 30 min bei Raumtemperatur gerührt. Anschließend wurde auf -70 °C gekühlt, eine Mischung aus Triethylamin (5,10 mmol) und Trifluorphenol (4,25 mmol als 90%ige Lösung in Toluol) in 3 ml Dichlormethan hinzugegeben und 1 h bei -70 °C gerührt. Dann wurde mit Triethylamintrishydrofluorid (14,18 mmol) versetzt und nach 5 min DBH (14,18 mmol), suspendiert in 15 ml Dichlormethan, portionsweise innerhalb von ca. 30 min hinzugegeben. Man ließ noch 60 min rühren, ließ den Ansatz auf -20 °C erwärmen und gab die orangefarbene Lösung unter Rühren auf 50 ml eiskalte 1 molare Natronlauge. Die organische Phase wurde abgetrennt und die wässrige Phase dreimal mit Dichlormethan extrahiert. Die vereinigten organischen Phasen wurden filtriert, zweimal mit gesättigter Natriumchloridlösung gewaschen und über Natriumsulfat getrocknet. Das Lösungsmittel wurde im Vakuum entfernt und der Rückstand mit n-Hexan über Kieselgel filtriert. Man erhielt ein farbloses Öl, das langsam durchkristallisierte.

### 2. Herstellung der Verbindung V.2

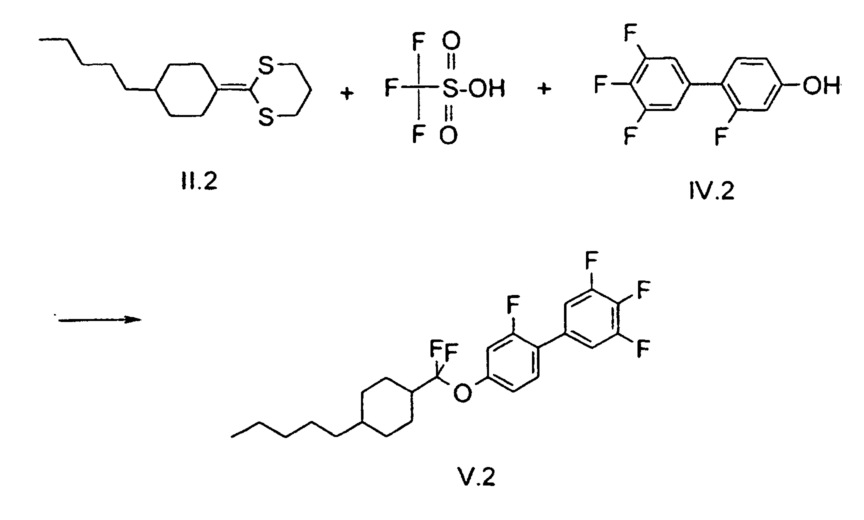

5,00 mmol des Ketendithioketal II.2 wurden in 50 ml Dichlormethan gelöst und unter Eiskühlung 5,00 mmol Trifluormethansulfonsäure zugetropft. Nach 15 min wurde die Kühlung entfernt und 30 min bei Raumtemperatur gerührt. Anschließend wurde auf -70 °C gekühlt, eine Mischung aus Triethylamin (9,00 mmol) und 7,50 mmol der Phenol-Verbindung IV.6 in 10 ml Dichlormethan hinzugegeben und 1 h bei -70 °C gerührt. Dann wurde mit Triethylamin-trishydrofluorid (25,00 mmol) versetzt und nach 5 min DBH (25,00 mmol), suspendiert in 15 ml Dichlormethan, portionsweise innerhalb von etwa 20 min hinzugegeben. Man ließ noch 60 min rühren, ließ den Ansatz auf -20 °C erwärmen und gab die orangefarbene Lösung unter Rühren auf 100 ml eiskalte 1 molare Natronlauge. Die organische Phase wurde abgetrennt und die wässrige Phase dreimal mit Dichlormethan extrahiert. Die vereinigten organischen Phasen wurden filtriert, zweimal mit gesättigter Natriumchloridlösung gewaschen und über Natriumsulfat getrocknet. Das Lösungsmittel wurde im Vakuum entfernt. Der Rest wurde aus Heptan bei -60 °C umkristallisiert.

### 3. Herstellung der Verbindung V.3

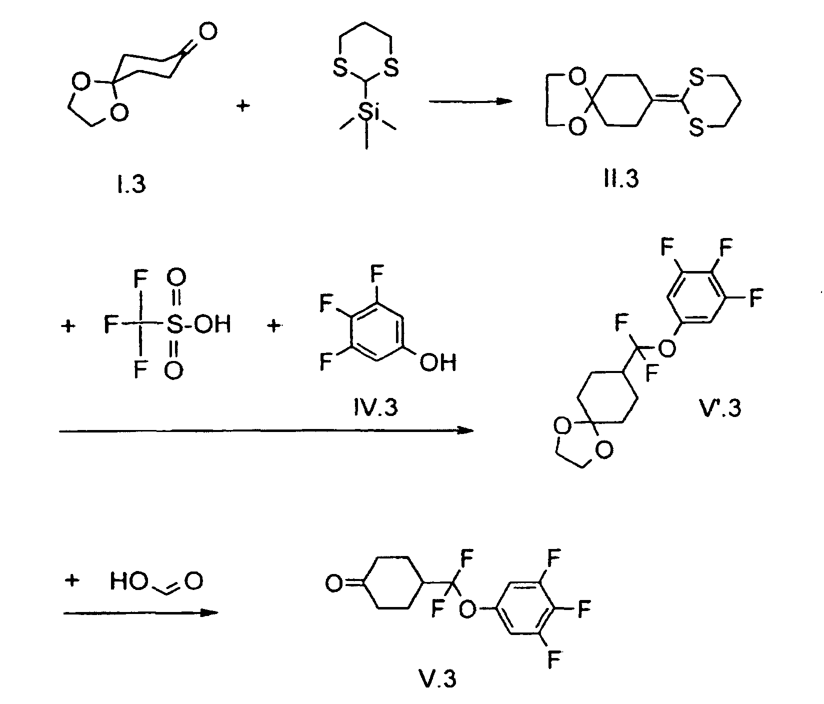

### 3.1 Herstellung des Ketendithioketals 11.3

0,130 mol 2-Trimethylsilyl-1,3-dithian wurden in 400 ml THF vorgelegt und 0,130 mol Butyllithium (15%ige Lösung in n-Hexan) bei -70 °C zugetropft. Es wurde 1 Stunde bei -70 °C gerührt, langsam auf -15 °C ansteigen lassen. Wieder bei -70 °C wurden 0,130 mol des Cyclohexandion 1.3, dessen eine Carbonylfunktion mit 1,2-Ethandiol als Ketal geschützt war, gelöst in 100 ml THF, zugetropft. Das Kältebad wurde entfernt und über Nacht bei etwa 20 °C nachgerührt.

Der Ansatz wurde mit 200 ml NaHCO₃-Lösung hydrolisiert. Es wurden noch 700 ml MTB-Ether zugegeben und zweimal mit je 200 ml Wasser gewaschen. Die wässrigen Phasen wurden noch zweimal mit Ether extrahiert. Die vereinigten organischen Phasen wurden mit Natriumsulfat getrocknet, filtriert und eingeengt.
¹H-NMR (CDCl₃, 250 MHz, 20°C): δ = 3,95 (s, 4H), 2.87 (mc, 4H), 2,61 (mc, 4H), 2,17-2,08 (m, 2H), 1,68 (t, J = 6,6 Hz, 4H).

### 3.2 Herstellung des Ethers V'.3

0,093 mol Ketendithioketal II.3 wurden in 300 ml Dichlormethan gelöst und 0,093 mol Trifluormethansulfonsäure bei +5 °C unter Rühren hinzugegeben.

Es wurde auf etwa 20 °C ansteigen lassen und eine Stunde weiter gerührt. Nach Abkühlen auf -70 °C wurde eine Mischung aus Trifluorphenol IV.3 (0,140 mol) und 0,167 mol Triethylamin in 30 ml Dichlormethan zugetropft und eine weitere Stunde bei -70 °C gerührt. Anschließend wurden 0,465 mol Triethylamin-trishydrofluorid zugetropft. Nach etwa 10 Minuten wurden 0,465 mol DBH, suspendiert in 170 ml Dichlormethan, verteilt über eine Stunde hinzugegeben und eine Stunde bei -70 °C gerührt.

Das Reaktionsgemisch einer Temperatur von -20 °C wurde unter Rühren in 300 ml 1 molare NaOH-Lösung gegeben. Die wässrige Phase wurde abgetrennt und einmal mit Dichlormethan extrahiert. Die vereinigten organischen Phasen wurden mit Wasser gewaschen, mit Natriumsulfat getrocknet, zweimal filtriert und eingeengt. Es wurde aus Heptan umkristallisiert. Schmelzpunkt: 67°C.
¹⁹F-NMR (CDCl₃, 235 MHz, 20°C): δ = -78,4 (d, J = 8,3 Hz, 2F), -133,5 (mc, 2F), -164,9 (mc, 1F).

### 3.3 Herstellung des Ethers V.3

0,017 mol des Ethers V'.3, 100 ml Toluol und 1,1 mol 98-100%ige Ameisensäure wurden zusammen über Nacht gerührt. Die Ameisensäure wurde abgetrennt und noch zweimal mit Toluol extrahiert. Die vereinigten organischen Phasen wurden zweimal mit Wasser gewaschen, mit Natriumsulfat getrocknet, filtriert und eingeengt.
¹⁹F-NMR (CDCl₃, 235 MHz, 20°C): δ = -76,1 (d, J = 8,4 Hz, 2F), -131,2 (mc, 2F), -162,4 (mc, 1F).

### 4. Herstellung der Verbindung V.4

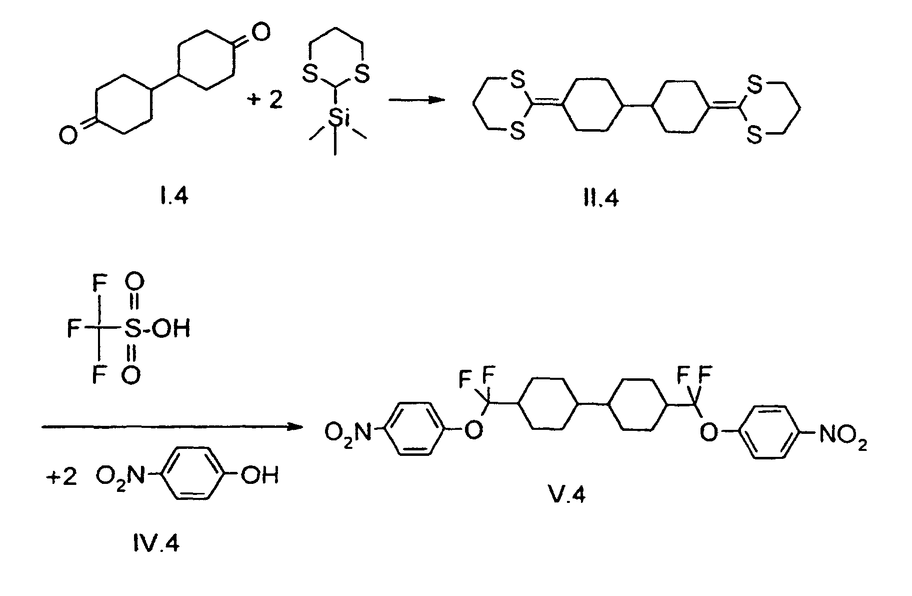

### 4.1 Herstellung des Ketendithioketals II.4

130 mmol 2-Trimethylsilyl-1,3-dithian wurden in 500 ml THF gelöst und bei -70 °C 130 mmol Butyllithium als 15%ige Lösung in n-Hexan zugetropft. Man ließ innerhalb von 4 h allmählich auf 0 °C erwärmen, kühlte erneut auf -70 °C und ließ das Keton 1.4 (60 mmol) in 100 ml THF zutropfen, wobei ein farbloser Niederschlag ausfiel. Nach beendeter Zugabe wurde die Kühlung entfernt und man ließ über Nacht rühren. Anschließend gab man den Ansatz auf 500 ml Eiswasser und extrahierte mit Dichlormethan, bis sämtlicher Niederschlag gelöst war. Die vereinigten organischen Phasen wurden zweimal mit gesättigter Natriumchloridlösung gewaschen und über Natriumsulfat getrocknet. Das Lösungsmittel wurde im Vakuum entfernt und das leicht gelbe Rohprodukt durch Umkristallisieren aus Dichlormethan gereinigt. Man erhielt farblose Blättchen.

### 4.2 Herstellung der Verbindung V.4

17,4 mmol des Ketendithioketal II.4 wurden in 100 ml Dichlormethan suspendiert und unter Eiskühlung 34,8 mmol Trifluormethansulfonsäure zutropfen gelassen. Anschließend ließ man die klare gelbe Lösung 1 h bei Raumtemperatur rühren. Dann wurde der Ansatz auf -70 °C abgekühlt und eine Lösung von 4-Nitrophenol IV.4 (52,2 mmol) und Triethylamin (62,6 mmol) in 20 ml Dichlormethan tropfenweise hinzugegeben. Nach 1 h wurde langsam mit Triethylamin-trishydrofluorid (152,0 mmol) versetzt und anschließend portionsweise innerhalb von 30 min eine Suspension von DBH (173,8 mmol) in 70 ml Dichlormethan hinzugegeben. Nach 60 min Rühren ließ man den Ansatz auf -20 °C erwärmen und gab die orangefarbene Suspension vorsichtig auf eine eiskalte Mischung aus 500 ml ca. 1 molare Natronlauge und 50 ml Natriumhydrogensulfitlösung. Der pH-Wert wurde mit 32 % Natronlauge auf 7 eingestellt, die wässrige Phase abgetrennt, dreimal mit Pentan extrahiert und die vereinigten organischen Phasen wurden über Natriumsulfat getrocknet. Das Lösungsmittel wurde im Vakuum entfernt und der Rückstand mit Dichlormethan/n-Heptan (1:1) über Kieselgel filtriert. Das Rohprodukt wurde aus Dichlormethan/n-Hexan umkristallisiert. Man erhielt schwach gelbe Kristalle (Schmelzpunkt: 164 °C).

### 5. Herstellung der Verbindung V.5

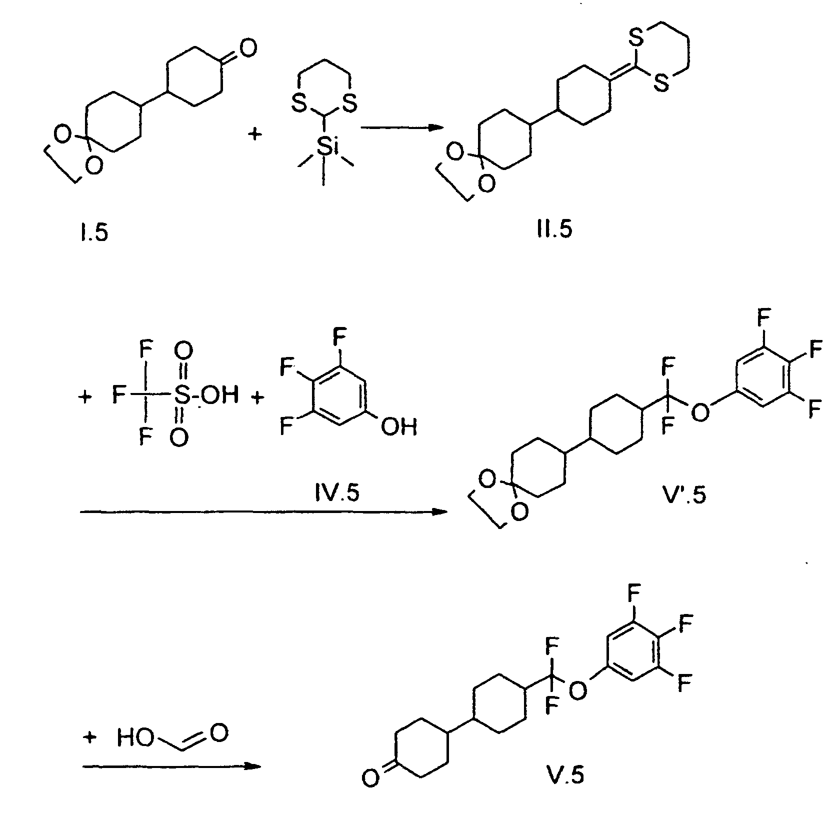

### 5.1 Herstellung des Ketendithioketal II.5

Zu einer Lösung von 2-Trimethylsilyl-1,3-dithian in 2,5 I THF wurden bei -70 °C 0,780 mol Butyllithium (als 15%ige Lösung in n-Hexan) zugetropft. Nach 30-minütigem weiterem Rühren bei -70 °C wurde langsam auf etwa -10 °C ansteigen lassen und noch 3 Stunden nachgerührt.

Wieder bei -70 °C wurde eine Lösung von 0,780 mol Bicyclohexanon 1.5, dessen eine Carbonylgruppe mit 1,2-Ethandiol als Ketal geschützt war, in 500 ml THF zugetropft. Über Nacht wurde bei etwa 20 °C gerührt. Das Reaktionsgemisch wurde mit 2 I Methyl-tert.-butylether und 500 ml NaHCO₃-Lösung versetzt. Es wurde wie üblich aufgearbeitet. Schmelzpunkt der Verbindung II.5: 129°C.

### 5.2 Herstellung des Ethers V'.5

0,100 mol Ketendithioketal IIb1.5 wurden in 300 ml Dichlormethan gelöst und 0,100 mol Trifluormethansulfonsäure bei +5 °C unter Rühren hinzugeben. Es wurden zwei weitere Stunden bei etwa 20 °C gerührt. Bei -70 °C wurde eine Mischung aus Trifluorphenol IV.5 (0,150 mol) und 0,180 mol Triethylamin in 30 ml Dichlormethan zugetropft und eine Stunde gerührt.

Anschließend wurden 0,500 mol Triethylamin-trishydrofluorid zugetropft und nach 10 Minuten wurden 0,500 mol DBH, suspendiert in 170 ml Dichlormethan, verteilt über 1 Stunde hinzugegeben, eine weitere Stunde bei -70 °C gerührt und das Reaktionsgemisch mit einer Temperatur von etwa -20 °C unter Rühren in 300 ml 1 molare NaOH-Lösung gegeben. Es wurde wie üblich aufgearbeitet.

### 5.3 Spaltung des Ketals unter Erhalt des Ethers V.5

0,083 mol des Ketals V'.5, gelöst in 250 ml Toluol, wurden mit 200 ml reiner Ameisensäure versetzt. Das Reaktionsgemisch wurde über Nacht bei etwa 20 °C gerührt. Anschließend wurde die Ameisensäure abgetrennt und diese zweimal mit Toluol extrahiert. Die vereinigten organischen Phasen wurden wie üblich aufgearbeitet.
¹⁹F-NMR (CDCl₃, 235 MHz, 20°C): δ = -79.4 (d, J = 8,4 Hz, 2F), -133,8 (mc, 2F), -165,2 (mc, 1F).

### 6. Synthese des Ethers V.6

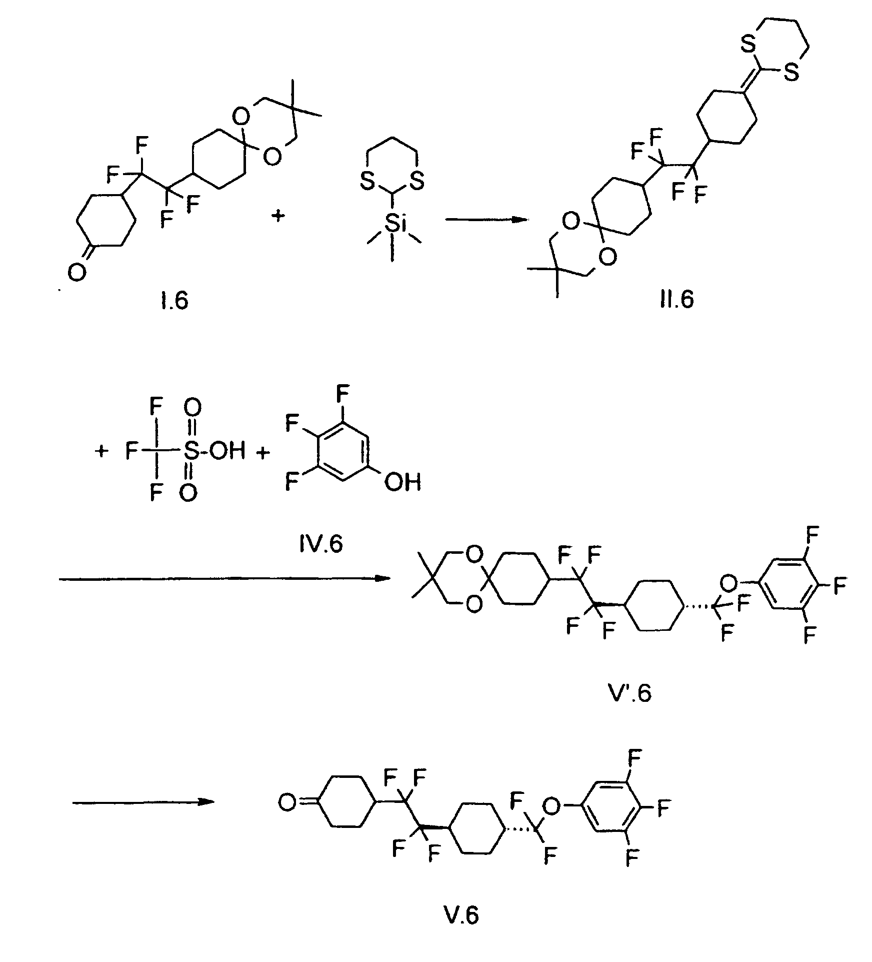

### 6.1 Herstellung des Ketendithioketals II.6

Zu 0,129 mol 2-Trimethylsilyl-1,3-dithian in 400 ml THF wurden bei -70 °C 0,131 mol n-Butyllithium als 15%ige Lösung in n-Hexan zugetropft und anschließend 30 Minuten bei -70 °C gerührt. Die Temperatur des Reaktionsgemisches wurde langsam auf -15 bis -5 °C erhöht und bei dieser Temperatur 3 Stunden gerührt. Wieder bei -70 °C wurden 0,128 mol der als Ketal einseitig geschützten Dicarbonylverbindung 1.6, gelöst in 100 ml THF, zugetropft. Anschließend wurde die Temperatur langsam auf etwa 20 °C erhöht und über Nacht gerührt. Das Reaktionsgemisch wurde mit 500 ml Methyl-tert.-butylether und 250 ml wässriger NaHCO₃-Lösung versetzt und anschließend wie üblich aufgearbeitet.
¹⁹F-NMR (CDCl₃, 235 MHz, 20°C): δ = -115,9 (mc, 2F), -116,3 (mc, 2F).

### 6.2 Herstellung des Ethers V'.6

Bei etwa 0 °C wurden 13,1 mmol Trifluormethansulfonsäure zu 13,1 mmol Ketendithioketal II.6, gelöst in 60 ml Dichlormethan, getropft. Nach 15-minütigem Rühren wurden langsam auf 20 °C erwärmt und noch 60 min gerührt. Bei -70 °C wurde eine Mischung aus 23,6 mmol Triethylamin, 19,7 mmol Trifluorphenol IV.6 und 60 ml Dichlormethan hinzugegeben und eine Stunde gerührt. Anschließend wurde 65,5 mmol Triethylamin-trishydrofluorid versetzt und nach 5 min 3,4 ml Brom, gelöst in 30 ml Dichlormethan, innerhalb von 10 min hinzugegeben. Nach 60 min weiteren Rühren wurde langsam auf -20 °C erwärmt und das Reaktionsgemisch unter Rühren in eine Mischung aus 20 ml gesättigter Natriumhydrogensulfit-Lösung und 150 ml 2 molarer Natronlauge gegeben. Die Aufarbeitung erfolgte wie üblich.
¹⁹F-NMR (CDCl₃, 235 MHz, 20°C): δ = -78,4 (d, J = 8,2 Hz, 2F), -115,0 (mc, 2F), -115,8 (mc, 2F), -132,5 (mc, 2F), -163,9 (mc, 1F).

### 6.3 Spaltung des Ketals zum Produkt V.6

8,90 mmol des Ketals V'.6 in 30 ml Toluol wurden zusammen mit 3,8 ml reiner Ameisensäure etwa 48 Stunden gerührt. Anschließend wurde die Ameisensäure-Phase abgetrennt, mit 100 ml Wasser verdünnt und dreimal mit Toluol extrahiert. Das Produkt wurde aus den vereinigten Toluol-Phasen wie üblich erhalten.
¹⁹F-NMR (CDCl₃, 235 MHz, 20°C): δ = -81,1 (d, J = 8,2 Hz, 2F), -117,2 (mc, 2F), -118,1 (mc, 2F), -135,2 (mc, 2F), -166,5 (mc, 1F).

### 7. Synthese der Verbindung V.7

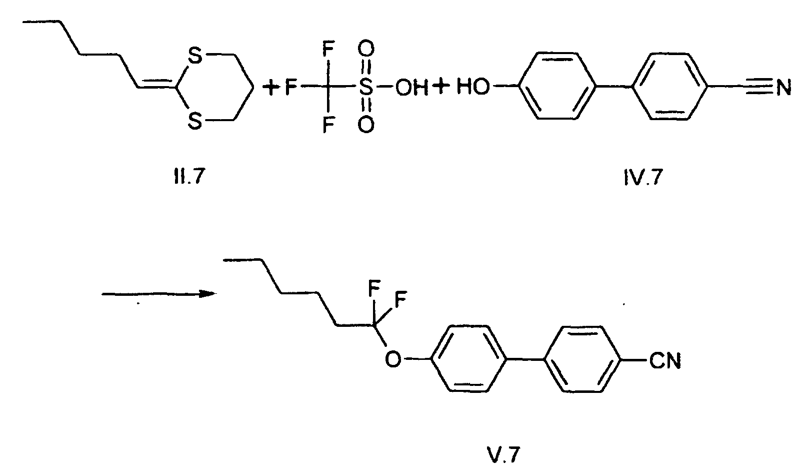

Zu einer Lösung von 19 mmol 2-Pentyliden-1,3-dithian 11.7 in 35 ml Dichlormethan wurden unter Eiskühlung 20 mmol Trifluormethansulfonsäure langsam zugegeben und 20 min gerührt. Anschließend wurde der Ansatz auf -70 C°abgekühlt und innerhalb von 15 min eine Lösung von 30 mmol Triethylamin und 4'-Hydroxybiphenyl-4-carbonsäurenitril (25 mmol) in 50 ml Dichlormethan zugetropft. Nach weiteren 45 min wurden langsam 96 mmol Triethylamin-trishydrofluorid hinzugegeben und dann innerhalb von 60 min portionsweise mit einer Suspension von 96 mmol DBH in 60 ml Dichlormethan versetzt. Nach 60 min Rühren wurde der Ansatz auf -20 C° erwärmt und die orangefarbene Suspension vorsichtig auf eine eiskalte Mischung aus 500 ml ca. 1 M Natronlauge und 50 ml Natriumhydrogensulfitlösung gegeben. Die wäßrige Phase wurde abgetrennt, dreimal mit Dichlormethan extrahiert und die vereinigten organischen Phasen wurden wie üblich aufgearbeitet (K 47 N 63 I, Δε = 17,7, Δn = 0,1844)
¹⁹F-NMR (CDCl₃, 235 MHz, 20°C): δ = -71.0 ppm (t, J = 11.3 Hz, 2F, -CF₂O-).
MS (EI): m/z (%) = 315 [M⁺] (33), 195 [M⁺ +H -C₅H₁₁CF₂] (100).

## Patentansprüche

1. Verfahren zur Herstellung von Verbindungen mit mindestens einer -CF₂-O-Brücke im Molekül, bei dem
a) an mindestens ein Ketendithioketal eine Säure addiert wird,
b) das erhaltene Bis(alkylthio)carbeniumsalz in Gegenwart einer Base mit mindestens einer mindestens eine Hydroxylgruppe aufweisenden Verbindung umgesetzt wird
c) und anschließend, vorzugsweise in situ, der erhaltene Dithioorthoester mit einem Fluorierungsmittel und einem Oxidationsmittel zur Verbindung mit mindestens einer -CF₂-O-Brücke im Molekül oxidativ fluorodesulfuriert wird.

2. Verfahren nach Anspruch 1, **gekennzeichnet durch** ein Ketendithioketal der Formel II 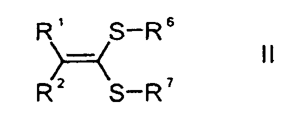 in der
R¹, R² unabhängig voneinander geradkettiges, verzweigtes oder cyclisches Alkyl mit 1 bis 25 C-Atomen, wobei R¹, R² derart miteinander verbrückt sein können, dass die Gruppe 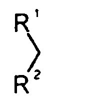 ein cyclisches Alkyl mit 4 bis 8 C-Atomen im Ring ist, und/oder worin ein oder mehrere H-Atome **durch** Halogen, -CN, weitere gegebenenfalls substituierte Alkyl- und/ oder Arylreste ersetzt sein können, und/ oder worin ein oder mehrere nicht benachbarte -CH₂-Gruppen unabhängig voneinander **durch** -CO-, -O-CO-, -CO-O-, -O-, -S-, -CH=CH-, -C≡C-, -NH- oder -N(CH₃)- ersetzt sein können, und/ oder
Aryl, das ein oder mehrfach mit Halogen, geradkettigem, verzweigtem und/ oder cyclischem Alkyl und/ oder Aryl, die ebenfalls substituiert sein können, substituiert sein kann,
worin auch ein oder mehrere CH-Gruppen **durch** N ersetzt sein können, bedeuten,
wobei einer der Reste R¹, R² auch H bedeuten kann,
R⁶, R⁷ unabhängig voneinander geradkettiges, verzweigtes oder cyclisches Alkyl mit 1 bis 12 C-Atomen, wobei R⁶, R⁷ derart miteinander verbrückt sein können, dass die Gruppe 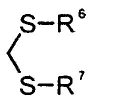 ein 4- bis 8-gliedriger Ring ist, und/ oder worin ein oder mehrere H-Atome **durch** Halogen, weitere gegebenenfalls substituierte Alkyl- und/ oder Arylreste ersetzt sein können, und/ oder worin ein oder mehrere nicht benachbarte -CH₂-Gruppen unabhängig voneinander **durch** -CO-, -O-, -S-, -CH=CH-, -C≡C-, -NH- oder -N(CH₃)- ersetzt sein können, und/ oder
Aryl, das ein oder mehrfach mit Halogen, geradkettigem, verzweigtem und/ oder cyclischem Alkyl und/ oder Aryl, das substituiert sein kann, bedeuten.

3. Verfahren nach Anspruch 1 oder 2, **dadurch gekennzeichnet, dass** die an das Ketendithioketal zu addierende Säure HY ist, wobei Y⁻ ein nicht- oder schwach-koordinierendes Anion ist.

4. Verfahren nach Anspruch 3, **dadurch gekennzeichnet, dass** Y⁻ ein Halogenid, Tetrafluoroborat, Hexafluorophosphat, Perchlorat oder Alkyl- oder Arylcarboxylat oder Alkyl- oder Arylsulfonat-Anion ist, wobei in den Alkyl- oder Arylgruppen ein, mehrere oder alle H-Atome durch Fluor oder Chlor substituiert sein können.

5. Verfahren nach einem der vorherigen Ansprüche, **gekennzeichnet durch** ein Bis(alkylthio)carbeniumsalz der Formel III 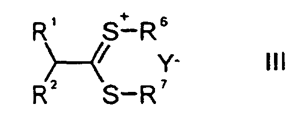 in der R¹, R², R⁶, R⁷ unabhängig voneinander eine der in Anspruch 2 angegebenen Bedeutungen aufweisen und Y⁻ eine der in Anspruch 3 oder 4 angegebenen Bedeutungen besitzt.

6. Verfahren nach einem der vorherigen Ansprüche, **dadurch gekennzeichnet, dass** die mindestens eine Hydroxylgruppe aufweisende Verbindung ein Alkyl- oder Arylalkohol der Formel IV R³-OH (IV) ist, in der der Alkyl- oder Arylrest R³ beliebig substituiert sein kann.

7. Verfahren nach einem der vorherigen Ansprüche, **gekennzeichnet durch** einen Dithioorthoester der Formel VI 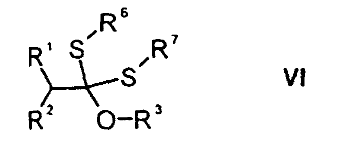 in der
R¹, R², R⁶, R⁷ unabhängig voneinander eine der in Anspruch 2 angegebenen Bedeutungen aufweisen und
R³ eine der in Anspruch 6 angegebenen Bedeutungen besitzt.

8. Verfahren nach einem der vorherigen Ansprüche, **dadurch gekennzeichnet, dass** das Oxidationsmittel eine Verbindung ist, die Haloniumäquivalente freisetzt, insbesondere ausgewählt ist aus der Gruppe, die gebildet ist von Dimethyldibromhydanthoin, N-Chlorsuccinimid, N-Bromsuccinimid, N-Jodsuccinimid, Dibromisocyanursäure, Chlor, Brom, SO₂Cl₂, SO₂ClF, Nitrosonium- und Nitroniumsalze, organische und anorganische Nitrite sowie Chloramin T.

9. Verfahren nach einem der vorherigen Ansprüche, **dadurch gekennzeichnet, dass** das Fluorierungsmittel ausgewählt ist aus der Gruppe, die gebildet ist von Fluorwasserstoff, aliphatischen und aromatischen Amin-Fluorwasserstoff-Komplexen, insbesondere ausgewählt ist aus der Gruppe, die gebildet ist von Pyridin-, Triethylamin-, Melamin-, Polyvinylpyridin-Fluorwasserstoff-Komplexen.

10. Verfahren nach einem der vorherigen Ansprüche, **gekennzeichnet durch** eine mindestens eine Hydroxylgruppe aufweisende Verbindung der Formel IVa 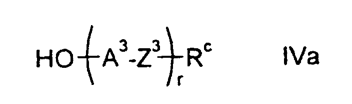 in der
R^{c} H, Halogen, -CN, -NCS, -SF₅ oder Alkyl mit 1 bis 18 C-Atomen, worin auch eine oder zwei nicht benachbarte -CH₂-Gruppen **durch** -O-, -S-, -CO-, -O-CO-, -CO-O-, -E- und/ oder -C≡C- ersetzt sein können und/ oder worin auch eine oder mehrere H-Atome **durch** Halogen und/ oder -CN ersetzt sein können, bedeutet,
E CR⁴=CR⁵ oder CHR⁴-CHR⁵,
R⁴, R⁵ jeweils unabhängig voneinander H, Alkyl mit 1-6 C-Atomen, F, Cl, Br, CF₃ oder CN bedeutet,
Z³ -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C=C- oder eine Einfachbindung,
A³ 1,4-Phenylen, worin ein oder mehrere CH-Gruppen **durch** N ersetzt sein können, 1,4-Cyclohexylen, worin ein oder zwei nicht benachbarte CH₂-Gruppen **durch** O und/ oder S ersetzt sein können, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei in diesen Gruppen ein oder mehrere H-Atome substituiert sein können **durch** Halogen, -CN und/ oder Alkyl mit 1 bis 6 C-Atomen, worin ein oder mehrere H-Atome **durch** Halogen oder -CN, und/ oder worin ein oder mehrere nicht benachbarte -CH₂-Gruppen unabhängig voneinander **durch** -CO-, -O-CO-, -CO-O-, -O-, -S-, -CH=CH-, -C≡C-, -NH- oder -N(CH₃)- ersetzt sein können, und
r 0, 1 oder 2 ist,
mit der Maßgabe, dass im Falle r = 0 R^{c} die angegebene Bedeutung Alkyl hat, bei der das C-Atom in 1-Position jedoch nicht **durch** ein Heteroatom ersetzt ist.

11. Verfahren nach einem der vorherigen Ansprüche, **dadurch gekennzeichnet, dass** die mindestens eine Hydroxylgruppe aufweisende Verbindung zwei Hydroxylgruppen aufweist.

12. Verfahren nach Anspruch 11, **gekennzeichnet durch** eine zwei Hydroxylgruppen aufweisende Verbindung der Formel IVd 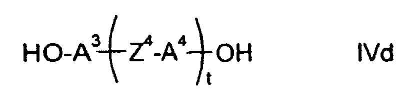 in der
A³ eine der in Anspruch 10 angegebenen Bedeutungen aufweist,
A⁴ eine der für A³ angegebenen Bedeutungen aufweist und
Z⁴ eine der in Anspruch 10 für Z³ angegebenen Bedeutungen besitzt und
t 0, 1 oder 2 ist.

13. Verfahren nach einem der vorherigen Ansprüche, **dadurch gekennzeichnet, dass** das Ketendithioketal aus einer Carbonylverbindung erhalten wird

14. Verfahren nach Anspruch 13, **gekennzeichnet durch** eine Carbonylverbindung der Formel I 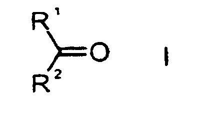 in der R¹ und R² unabhängig voneinander eine der in Anspruch 2 angegebenen Bedeutungen aufweisen.

15. Verfahren nach Anspruch 13 oder 14, **gekennzeichnet durch** eine Carbonylverbindung der Formel la 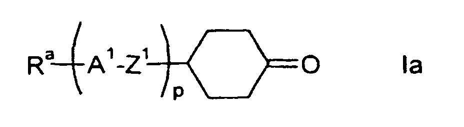 in der
R^{a} H, Halogen, -CN oder Alkyl mit 1 bis 18 C-Atomen, worin auch eine oder zwei nicht benachbarte -CH₂-Gruppen **durch** -O-, -S-, -CO-, -O-CO-, -CO-O-, -E- und/ oder -C≡C- ersetzt sein können und/ oder worin auch ein oder mehrere H-Atome **durch** Halogen und/ oder CN ersetzt sein können, bedeutet,
E CR⁴=CR⁵ oder CHR⁴-CHR⁵,
R⁴, R⁵ jeweils unabhängig voneinander H, Alkyl mit 1-6 C-Atomen, F, Cl, Br, CF₃ oder CN bedeutet und
Z¹ -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- oder eine Einfachbindung, und
A¹ 1,4-Phenylen, worin ein oder mehrere CH-Gruppen **durch** N ersetzt sein können, 1,4-Cyclohexylen, worin ein oder zwei nicht benachbarte CH₂-Gruppen **durch** O und/ oder S ersetzt sein können, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei in diesen Gruppen ein oder mehrere H-Atome substituiert sein können **durch** Halogen, -CN und/ oder Alkyl mit 1 bis 6 C-Atomen, worin ein oder mehrere H-Atome **durch** Halogen oder -CN, und/oder worin ein oder mehrere nicht benachbarte -CH₂-Gruppen unabhängig voneinander **durch** -CO-, -O-CO-, -CO-O-, -O-, -S-, -CH=CH-, -C≡C-, -NH- oder -N(CH₃)- ersetzt sein können, und
p 0, 1 oder 2 ist.

16. Verfahren nach einem der vorherigen Ansprüche, **dadurch gekennzeichnet, dass** die Carbonylverbindung zwei oder mehr Carbonylgruppen aufweist.

17. Verfahren nach Anspruch 16, **dadurch gekennzeichnet, dass** die Carbonylverbindung mit zwei oder mehr Carbonylguppen ein Cyclohexandion der Formel Ib 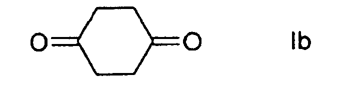 oder eine Verbindung der Formel Ic 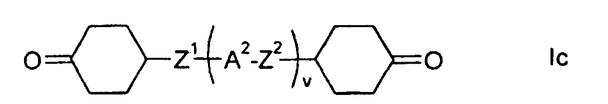 in der
Z¹, Z² jeweils unabhängig voneinander eine der in Anspruch 15 für Z¹ angegebenen Bedeutungen aufweisen,
A² eine der in Anspruch 15 für A¹ angebenen Bedeutungen besitzt, und
v 0, 1 oder 2 ist.

18. Verfahren nach Anspruch 16 oder 17, **dadurch gekennzeichnet, dass** eine oder mehrere Carbonylgruppen der Carbonylverbindung vor der Umsetzung zum entsprechenden Ketendithioketal als Ketal geschützt werden, wobei mindestens eine Carbonylgruppe zur Umsetzung zum Ketendithioketal ungeschützt bleibt.

19. Verfahren nach einem der Ansprüche 13 bis 18, **dadurch gekennzeichnet, dass** das Ketendithioketal aus der Carbonylverbindung durch Umsetzung mit gegebenenfalls substituiertem 2-Silyl-1,3-dithian erhalten wird.

20. Ketendithioketal der Formel IIa 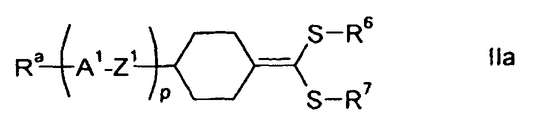 und/ oder das entsprechende Bis(alkylthio)carbeniumsalz der Formel IIIa 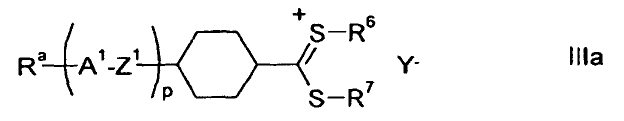 in denen
R^{a} H, Halogen, -CN oder Alkyl mit 1 bis 18 C-Atomen, worin auch eine oder zwei nicht benachbarte -CH₂-Gruppen durch -O-, -S-, -CO-, -O-CO-, -CO-O-, -E- und/ oder -C≡C- ersetzt sein können und/ oder worin auch eine oder mehrere H-Atome durch Halogen und/ oder CN ersetzt sein können, bedeutet,
E CR⁴=CR⁵ oder CHR⁴-CHR⁵,
R⁴, R⁵ jeweils unabhängig voneinander H, Alkyl mit 1-6 C-Atomen, F, Cl, Br, CF₃ oder CN bedeutet und
Z¹ -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C=C- oder eine Einfachbindung,
A¹ 1,4-Phenylen, worin ein oder mehrere CH-Gruppen durch N ersetzt sein können, 1,4-Cyclohexylen, worin ein oder zwei nicht benachbarte CH₂-Gruppen durch O und/ oder S ersetzt sein können, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei in diesen Gruppen ein oder mehrere H-Atome substituiert sein können durch Halogen, -CN und/ oder Alkyl mit 1 bis 6 C-Atomen, worin ein oder mehrere H-Atome durch Halogen oder -CN, und/oder worin ein oder mehrere nicht benachbarte -CH₂-Gruppen unabhängig voneinander durch -CO-, -O-CO-, -CO-O-, -O-, -S-, -CH=CH-, -C≡C-, -NH- oder -N(CH₃)- ersetzt sein können,
p 0, 1 oder 2 ist, und
R⁶, R⁷ unabhängig voneinander geradkettiges, verzweigtes oder cyclisches Alkyl mit 1 bis 12 C-Atomen, worin ein- oder mehrere H-Atome durch Halogen, weitere gegebenenfalls substituierte Alkyl- und/ oder Arylreste ersetzt sein können, und/ oder worin ein oder mehrere nicht benachbarte -CH₂-Gruppen unabhängig voneinander durch -CO-, -O-, -S-, -CH=CH-, -C≡C-, -NH- oder -N(CH₃)- ersetzt sein können, und/ oder
Aryl, das ein oder mehrfach mit Halogen, geradkettigem, verzweigtem und/ oder cyclischem Alkyl und/ oder Aryl, das substituiert sein kann, bedeutet und
Y⁻ ein nicht- oder schwach-koordinierendes Anion ist.

21. Ketendithioketal der Formel IIb1 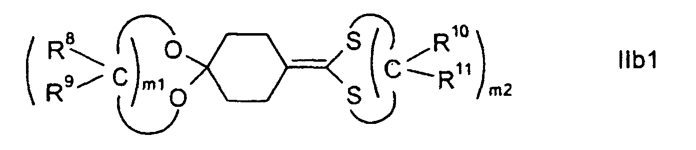 und/ oder das entsprechende Bis(alkylthio)carbeniumsalz der Formel IIIb1 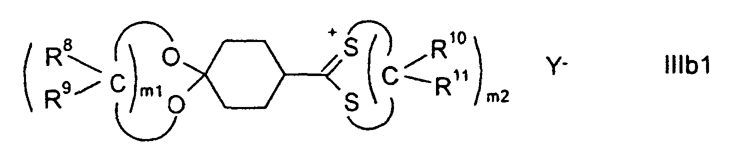 in denen
R⁸, R⁹, R¹⁰, R¹¹ jeweils unabhängig voneinander H oder eine gegebenenfalls substituierte Alkyl- oder Alkenylgruppe mit 1 bis 6 C-Atomen bedeutet, wobei jeweils eine oder beide Gruppen 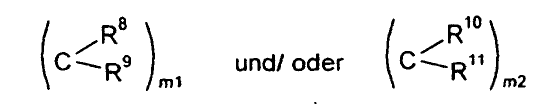 eine Cycloalkyl oder Arylgruppe ausbilden können, und
m1, m2 2, 3 oder 4 ist und
Y⁻ ein nicht- oder schwach-koordinierendes Anion ist.

22. Verbindung der Formel Vb 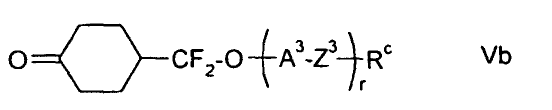 und/ oder das entsprechende Ketal der Formel Vb1 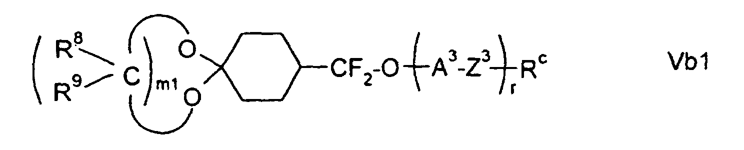 in denen
R⁸, R⁹ und m1 eine der entsprechenden, in Anspruch 21 angegebenen Bedeutungen besitzen und
R^{c} H, Halogen, -CN, -NCS, -SF₅ oder Alkyl mit 1 bis 18 C-Atomen, worin auch eine oder zwei nicht benachbarte -CH₂-Gruppen durch -O-, -S-, -CO-, -O-CO-, -CO-O-, -E- und/ oder -C≡C- ersetzt sein können und/ oder worin auch eine oder mehrere H-Atome durch Halogen und/ oder -CN ersetzt sein können, bedeutet,
E CR⁴=CR⁵ oder CHR⁴-CHR⁵,
R⁴, R⁵ jeweils unabhängig voneinander H, Alkyl mit 1-6 C-Atomen, F, Cl, Br, CF₃ oder CN bedeutet und
Z³ -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- oder eine Einfachbindung, und
A³ 1,4-Phenylen, worin ein oder mehrere CH-Gruppen durch N ersetzt sein können, 1,4-Cyclohexylen, worin ein oder zwei nicht benachbarte CH₂-Gruppen durch O und/ oder S ersetzt sein können, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei in diesen Gruppen ein oder mehrere H-Atome substituiert sein können durch Halogen, -CN und/ oder Alkyl mit 1 bis 6 C-Atomen, worin ein oder mehrere H-Atome durch Halogen oder -CN, und/ oder worin ein oder mehrere nicht benachbarte -CH₂-Gruppen unabhängig voneinander durch -CO-, -O-CO-, -CO-O-, -O-, -S-, -CH=CH-, -C≡C-, -NH- oder -N(CH₃)- ersetzt sein können, und
r 0, 1 oder 2 ist,
mit der Maßgabe, dass im Falle r = 0 R^{c} die angegebene Bedeutung Alkyl hat, bei der das C-Atom in 1-Position jedoch nicht durch ein Heteroatom substituiert ist.

23. Ketendithioketal der Formel IIb2 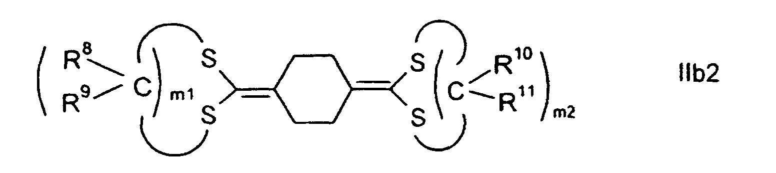 und/ oder das entsprechende Bis(alkylthio)carbeniumsalz der Formel IIIb2 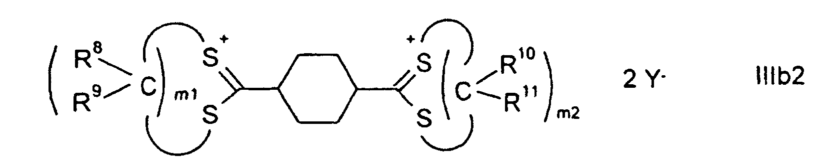 in denen
R⁸, R⁹, R¹⁰, R¹¹, m1 und m2 jeweils eine der entsprechenden, in Anspruch 21 angegebenen Bedeutungen besitzen und
Y⁻ ein nicht- oder schwach-koordinierendes Anion ist.

24. Ketendithioketal der Formel IIc1 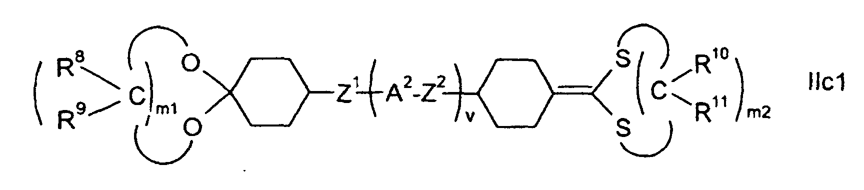 und/ oder das entsprechende Bis(alkylthio)carbeniumsalz der Formel IIIc1 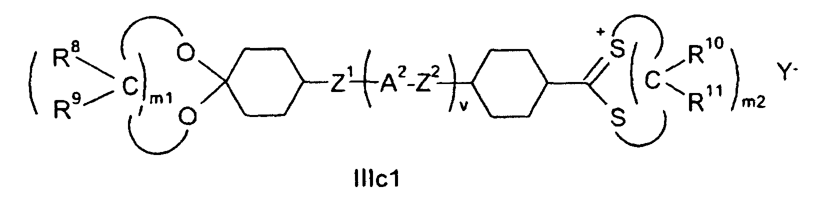 in denen
R⁸, R⁹, R¹⁰, R¹¹, m1 und m2 eine der entsprechenden, in Anspruch 21 angegebenen Bedeutungen besitzen,
Z¹ eine der entsprechenden, in Anspruch 20 angegebenen Bedeutungen besitzt und
A² und Z² eine der A¹ bzw. Z¹ entsprechenden, in Anspruch 20 angegebenen Bedeutungen hat,
V 0, 1 oder 2 ist und
Y⁻ ein nicht- oder schwach-koordinierendes Anion ist.

25. Verbindung der Formel Vc 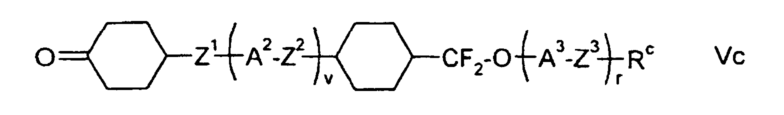 und/ oder das entsprechende Ketal der Formel Vc1 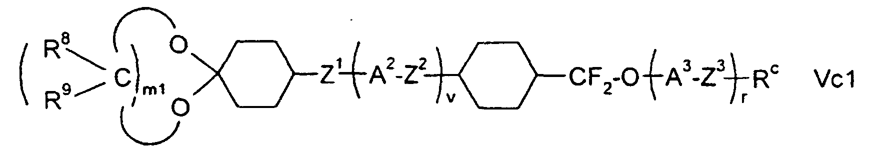 in denen
R⁸, R⁹, m1, Z¹, A², Z² und v eine der entsprechenden, in Anspruch 24 angegebenen Bedeutungen und
A³, Z³, r und R^{c} jeweils eine der entsprechenden, in Anspruch 22 angegebenen Bedeutungen besitzen.

26. Ketendithioketal der Formel IIc2 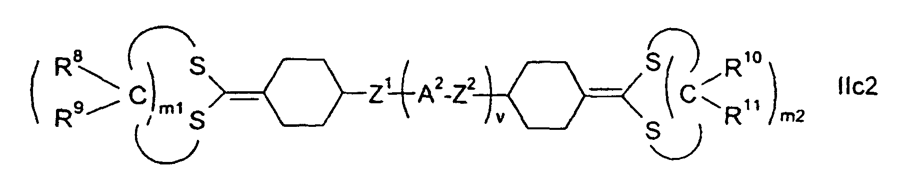 und das entsprechende Bis(alkylthio)carbeniumsalz der Formel, IIIc2 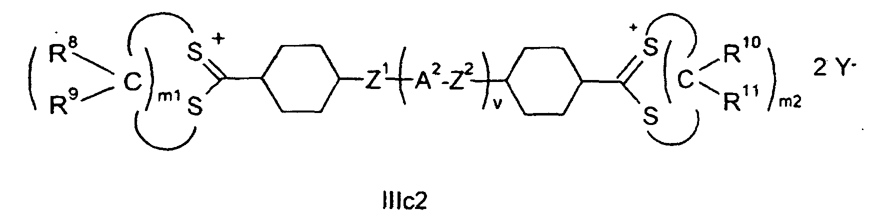 in denen
R⁸, R⁹, R¹⁰, R¹¹, m1, m2, Z¹, A², Z² und v eine der entsprechenden, in Anspruch 24 angegebenen Bedeutungen besitzen und
Y⁻ ein nicht- oder schwach-koordinierendes Anion ist.

27. Diketon der Formel Ic.1 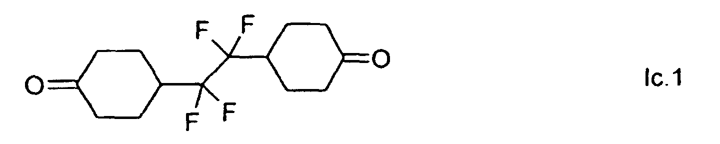 und/ oder das entsprechende Ketal der Formel Ic1.1 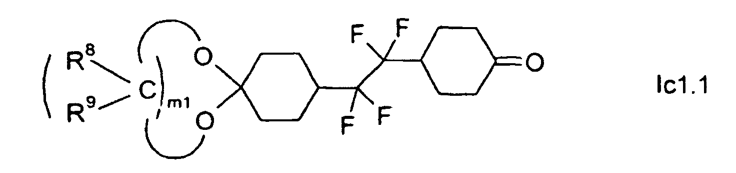 in der
R⁸, R⁹ und m1 eine der entsprechenden, in Anspruch 22 angegebenen Bedeutungen besitzen.

## Claims

1. Process for the preparation of compounds containing at least one -CF₂-O- bridge in the molecule, in which
a) an acid is added onto at least one ketene dithioketal,
b) the resultant bis(alkylthio)carbenium salt is reacted with at least one compound contaning at least one hydroxyl group in the presence of a base,
c) and subsequently, preferably in situ, the resultant dithioorthoester is subjected to oxidative fluorodesulfurisation using a fluorinating agent and an oxidant to give the compound containing at least one -CF₂-O- bridge in the molecule.

2. Process according to Claim 1, **characterised by** a ketene dithioketal of the formula II 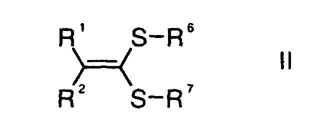 in which
R¹, R², independently of one another, denote straight-chain, branched or cyclic alkyl having 1 to 25 C atoms, where R¹, R² may be bridged with one another in such a way that the 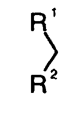 group is a cyclic alkyl having 4 to 8 C atoms in the ring, and/or in which one or more H atoms may be replaced by halogen, -CN, further optionally substituted alkyl and/or aryl radicals, and/or in which one or more non-adjacent -CH₂- groups may be replaced, independently of one another, by -CO-, -O-CO-, -CO-O-, -O-, -S-, -CH=CH-, -C≡C-, -NH- or -N(CH₃)-, and/or
aryl, which may be mono- or polysubstituted by halogen, straight-chain, branched and/or cyclic alkyl and/or aryl, which may likewise be substituted, in which, in addition, one or more CH groups may be replaced by N,
where one of the radicals R¹, R² may alternatively denote H,
R⁶, R⁷, independently of one another, denote straight-chain, branched or cyclic alkyl having 1 to 12 C atoms, where R⁶, R⁷ may be bridged with one another in such a way that the 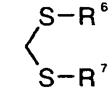 group is a 4- to 8-membered ring, and/or in which one or more H atoms may be replaced by halogen, further optionally substituted alkyl and/or aryl radicals, and/or in which one or more non-adjacent -CH₂- groups may be replaced, independently of one another, by -CO-, -O-, -S-, -CH=CH-, -C≡C-, -NH- or -N(CH₃)-, and/or
aryl, which may be mono- or polysubstituted by halogen or straight-chain, branched and/or cyclic alkyl and/or aryl.

3. Process according to Claim 1 or 2, **characterised in that** the acid to be added onto the ketene dithioketal is HY, in which Y⁻ is a non-coordinating or weakly coordinating anion.

4. Process according to Claim 3, **characterised in that** Y⁻ is a halide, tetrafluoroborate, hexafluorophosphate, perchlorate or alkyl- or aryl-carboxylate or alkyl- or arylsulfonate anion, where one, a plurality or all of the H atoms in the alkyl or aryl groups may be substituted by fluorine or chlorine.

5. Process according to one of the preceding claims, **characterised by** a bis(alkylthio)carbenium salt of the formula III 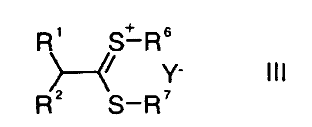 in which R¹, R², R⁶, R⁷, independently of one another, have one of the meanings indicated in Claim 2, and Y⁻ has one of the meanings indicated in Claim 3 or 4.

6. Process according to one of the preceding claims, **characterised in that** the compound containing at least one hydroxyl group is an alkyl or aryl alcohol of the formula IV R³-OH (IV), in which the alkyl or aryl radical R³ may be substituted as desired.

7. Process according to one of the preceding claims, **characterised by** a dithioorthoester of the formula VI 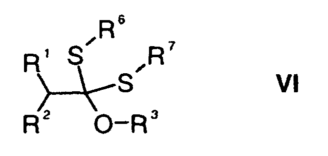 in which
R¹, R², R⁶, R⁷, independently of one another, have one of the meanings indicated in Claim 2, and
R³ has one of the meanings indicated in Claim 6.

8. Process according to one of the preceding claims, **characterised in that** the oxidant is a compound which liberates halonium equivalents, in particular is selected from the group formed by dimethyldibromohydantoin, N-chlorosuccinimide, N-bromosuccinimide, N-iodosuccinimide, dibromoisocyanuric acid, chlorine, bromine, SO₂Cl₂, SO₂CIF, nitrosonium and nitronium salts, organic and inorganic nitrites and chloramine T.

9. Process according to one of the preceding claims, **characterised in that** the fluorinating agent is selected from the group formed by hydrogen fluoride, aliphatic and aromatic amine/hydrogen fluoride complexes, in particular selected from the group formed by pyridine/, triethylamine/, melamine/, polyvinylpyridine/hydrogen fluoride complexes.

10. Process according to one of the preceding claims, **characterised by** a compound containing at least one hydroxyl group, of the formula IVa 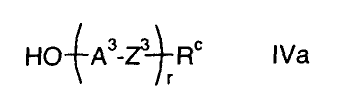 in which
R^{c} denotes H, halogen, -CN, -NCS, -SF₅ or alkyl having 1 to 18 C atoms, in which, in addition, one or two non-adjacent -CH₂- groups may be replaced by -O-, -S-, -CO-, -O-CO-, -CO-O-, -E- and/or -C≡C-, and/or in which, in addition, one or more H atoms may be replaced by halogen and/or -CN,
E denotes CR⁴=CR⁵ or CHR⁴-CHR⁵,
R⁴, R⁵ each, independently of one another, denote H, alkyl having 1-6 C atoms, F, Cl, Br, CF₃ or CN,
Z³ is -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- or a single bond,
A³ is 1,4-phenylene, in which one or more CH groups may be replaced by N, 1,4-cyclohexylene, in which one or two non-adjacent CH₂ groups may be replaced by O and/or S, 1,4-cyclohexenylene, 1,4-bicyclo[2.2.2]octylene, piperidine-1,4-diyl, naphthalene-2,6-diyl, decahydronaphthalene-2,6-diyl or 1,2,3,4-tetrahydronaphthalene-2,6-diyl, where one or more H atoms in these groups may be substituted by halogen, -CN and/or alkyl having 1 to 6 C atoms, in which one or more H atoms may be replaced by halogen or -CN, and/or in which one or more non-adjacent -CH₂- groups may be replaced, independently of one another, by -CO-, -O-CO-, -CO-O-, -O-, -S-, -CH=CH-, -C≡C-, -NH- or -N(CH₃)-, and
r is 0, 1 or 2,
with the proviso that, in the case where r = 0, R^{c} has the indicated meaning of alkyl, but in which the C atom in the 1-position has not been replaced by a heteroatom.

11. Process according to one of the preceding claims, **characterised in that** the compound containing at least one hydroxyl group contains two hydroxyl groups.

12. Process according to Claim 11, **characterised by** a compound containing two hydroxyl groups, of the formula IVd 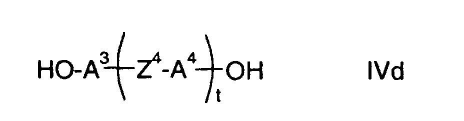 in which
A³ has one of the meanings indicated in Claim 10,
A⁴ has one of the meanings indicated for A³, and
Z⁴ has one of the meanings indicated for Z³ in Claim 10, and
t is 0, 1 or 2.

13. Process according to one of the preceding claims, **characterised in that** the ketene dithioketal is obtained from a carbonyl compound.

14. Process according to Claim 13, **characterised by** a carbonyl compound of the formula I 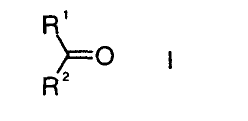 in which R¹ and R², independently of one another, have one of the meanings indicated in Claim 2.

15. Process according to Claim 13 or 14, **characterised by** a carbonyl compound of the formula la 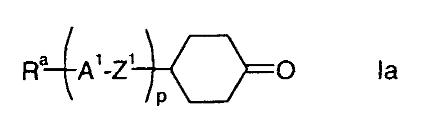 in which
R^{a} denotes H, halogen, -CN or alkyl having 1 to 18 C atoms, in which, in addition, one or two non-adjacent -CH₂- groups may be replaced by -O-, -S-, -CO-, -O-CO-, -CO-O-, -E- and/or -C≡C-, and/or in which, in addition, one or more H atoms may be replaced by halogen and/or CN,
E denotes CR⁴=CR⁵ or CHR⁴-CHR⁵,
R⁴, R⁵ each, independently of one another, denote H, alkyl having 1-6 C atoms, F, Cl, Br, CF₃ or CN, and
Z¹ is -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- or a single bond, and
A¹ is 1,4-phenylene, in which one or more CH groups may be replaced by N, 1,4-cyclohexylene, in which one or two non-adjacent CH₂ groups may be replaced by O and/or S, 1,4-cyclohexenylene, 1,4-bicyclo[2.2.2]octylene, piperidine-1,4-diyl, naphthalene-2,6-diyl, decahydronaphthalene-2,6-diyl or 1,2,3,4-tetrahydronaphthalene-2,6-diyl, where one or more H atoms in these groups may be substituted by halogen, -CN and/or alkyl having 1 to 6 C atoms, in which one or more H atoms may be replaced by halogen or -CN, and/or in which one or more non-adjacent -CH₂- groups may be replaced, independently of one another, by -CO-, -O-CO-, -CO-O-, -O-, -S-, -CH=CH-, -C≡C-, -NH- or -N(CH₃)-, and
p is 0, 1 or 2.

16. Process according to one of the preceding claims, **characterised in that** the carbonyl compound contains two or more carbonyl groups.

17. Process according to Claim 16, **characterised in that** the carbonyl compound containing two or more carbonyl groups is a cyclohexanedione of the formula Ib 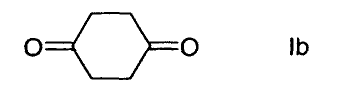 or a compound of the formula Ic 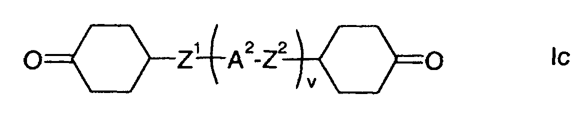 in which
Z¹, Z² each, independently of one another, have one of the meanings indicated for Z' in Claim 15,
A² has one of the meanings indicated for A¹ in Claim 15, and
v is 0, 1 or 2.

18. Process according to Claim 16 or 17, **characterised in that** one or more carbonyl groups of the carbonyl compound are protected as a ketal before the conversion into the corresponding ketene dithioketal, with at least one carbonyl group remaining unprotected for conversion into the ketene dithioketal.

19. Process according to one of Claims 13 to 18, **characterised in that** the ketene dithioketal is obtained from the carbonyl compound by reaction with substituted or unsubstituted 2-silyl-1,3-dithiane.

20. Ketene dithioketal of the formula IIa 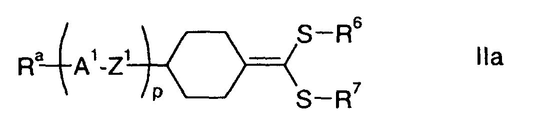 and/or the corresponding bis(alkylthio)carbenium salt of the formula IIIa 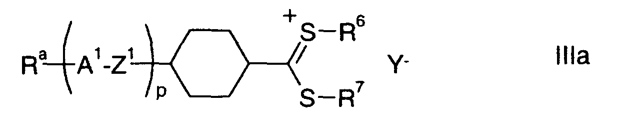 in which
R^{a} denotes H, halogen, -CN or alkyl having 1 to 18 C atoms, in which, in addition, one or two non-adjacent -CH₂- groups may be replaced by -O-, -S-, -CO-, -O-CO-, -CO-O-, -E- and/or -C≡C-, and/or in which, in addition, one or more H atoms may be replaced by halogen and/or CN,
E denotes CR⁴=CR⁵ or CHR⁴-CHR⁵,
R⁴, R⁵ each, independently of one another, denote H, alkyl having 1-6 C atoms, F, Cl, Br, CF₃ or CN, and
Z¹ is -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- or a single bond,
A¹ is 1,4-phenylene, in which one or more CH groups may be replaced by N, 1,4-cyclohexylene, in which one or two non-adjacent CH₂ groups may be replaced by O and/or S, 1,4-cyclohexenylene, 1,4-bicyclo[2.2.2]octylene, piperidine-1,4-diyl, naphthalene-2,6-diyl, decahydronaphthalene-2,6-diyl or 1,2,3,4-tetrahydronaphthalene-2,6-diyl, where one or more H atoms in these groups may be substituted by halogen, -CN and/or alkyl having 1 to 6 C atoms, in which one or more H atoms may be replaced by halogen or -CN, and/or in which one or more non-adjacent -CH₂- groups may be replaced, independently of one another, by -CO-, -O-CO-, -CO-O-, -O-, -S-, -CH=CH-, -C≡C-, -NH- or -N(CH₃)-,
p is 0, 1 or 2, and
R⁶, R⁷, independently of one another, denote straight-chain, branched or cyclic alkyl having 1 to 12 C atoms, in which one or more H atoms may be replaced by halogen, further optionally substituted alkyl and/or aryl radicals, and/or in which one or more non-adjacent -CH₂- groups may be replaced, independently of one another, by -CO-, -O-, -S-, -CH=CH-, -C≡C-, -NH- or -N(CH₃)-, and/or
aryl, which may be mono- or polysubstituted by halogen, straight-chain, branched and/or cyclic alkyl and/or aryl, and
Y⁻ is a non-coordinating or weakly coordinating anion.

21. Ketene dithioketal of the formula IIb1 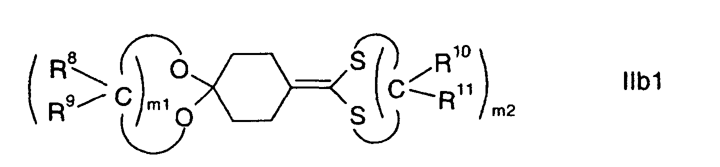 and/or the corresponding bis(alkylthio)carbenium salt of the formula IIIb1 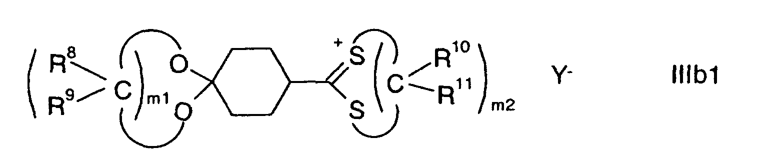 in which
R⁸, R⁹, R¹⁰, R¹¹ each, independently of one another, denote H or a substituted or unsubstituted alkyl or alkenyl group having 1 to 6 C atoms, where in each case one or both 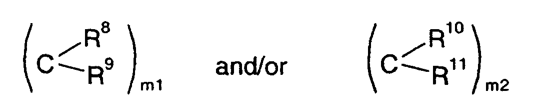 groups may form a cycloalkyl or aryl group, and
m1, m2 are 2, 3 or 4, and
Y⁻ is a non-coordinating or weakly coordinating anion.

22. Compound of the formula Vb 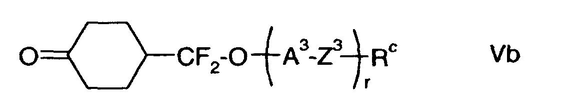 and/or the corresponding ketal of the formula Vb1 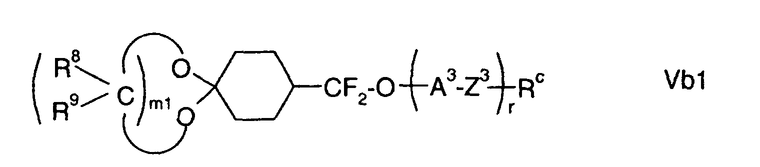 in which
R⁸, R⁹ and m1 have one of the corresponding meanings indicated in Claim 21, and
R^{c} denotes H, halogen, -CN, -NCS, -SF₅ or alkyl having 1 to 18 C atoms, in which, in addition, one or two non-adjacent -CH₂- groups may be replaced by -O-, -S-, -CO-, -O-CO-, -CO-O-, -E- and/or -C≡C-, and/or in which, in addition, one or more H atoms may be replaced by halogen and/or -CN,
E denotes CR⁴=CR⁵ or CHR⁴-CHR⁵,
R⁴, R⁵ each, independently of one another, denote H, alkyl having 1-6 C atoms, F, Cl, Br, CF₃ or CN, and
Z³ is -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- or a single bond, and
A³ is 1,4-phenylene, in which one or more CH groups may be replaced by N, 1,4-cyclohexylene, in which one or two non-adjacent CH₂ groups may be replaced by O and/or S, 1,4-cyclohexenylene, 1,4-bicyclo[2.2.2]octylene, piperidine-1,4-diyl, naphthalene-2,6-diyl, decahydronaphthalene-2,6-diyl or 1,2,3,4-tetrahydronaphthalene-2,6-diyl, where one or more H atoms in these groups may be substituted by halogen, -CN and/or alkyl having 1 to 6 C atoms, in which one or more H atoms may be replaced by halogen or -CN, and/or in which one or more non-adjacent -CH₂- groups may be replaced, independently of one another, by -CO-, -O-CO-, -CO-O-, -O-, -S-, -CH=CH-, -C≡C-, -NH- or -N(CH₃)-, and
r is 0, 1 or 2,
with the proviso that, in the case where r = 0, R^{c} has the indicated meaning of alkyl, but in which the C atom in the 1-position is not substituted by a heteroatom.

23. Ketene dithioketal of the formula IIb2 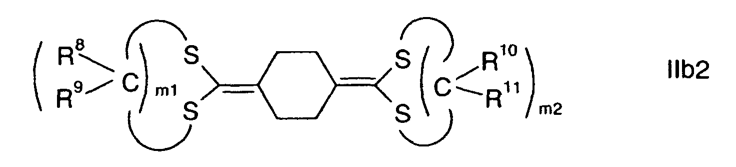 and/or the corresponding bis(alkylthio)carbenium salt of the formula IIIb2 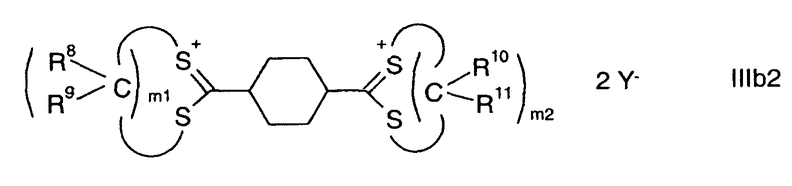 in which
R⁸, R⁹, R¹⁰, R¹¹, m1 and m2 each have one of the corresponding meanings indicated in Claim 21, and
Y⁻ is a non-coordinating or weakly coordinating anion.

24. Ketene dithioketal of the formula IIc1 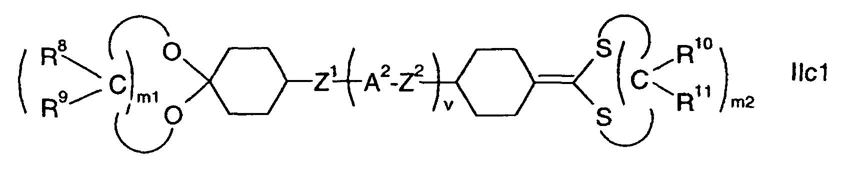 and/or the corresponding bis(alkylthio)carbenium salt of the formula IIIc1 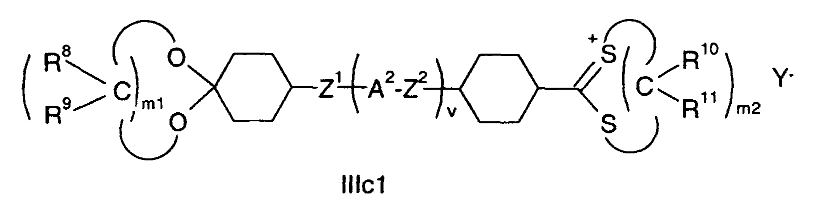 in which
R⁸, R⁹, R¹⁰, R¹¹, m1 and m2 have one of the corresponding meanings indicated in Claim 21,
Z¹ has one of the corresponding meanings indicated in Claim 20, and
A² and Z² have one of the meanings corresponding to A¹ and Z¹ respectively indicated in Claim 20,
v is 0, 1 or 2, and
Y is a non-coordinating or weakly coordinating anion.

25. Compound of the formula Vc 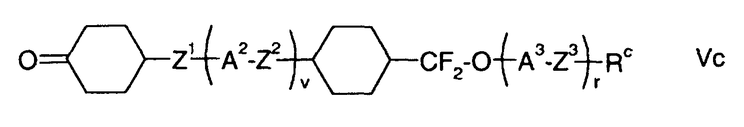 and/or the corresponding ketal of the formula Vc1 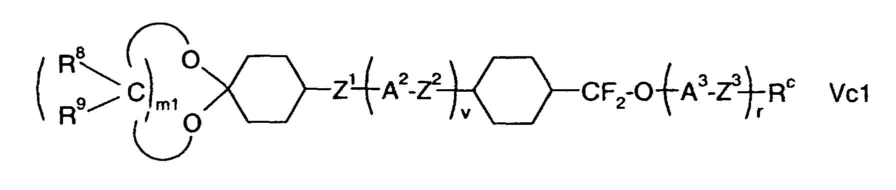 in which
R⁸, R⁹, m1, Z¹, A², Z² and v have one of the corresponding meanings indicated in Claim 24, and
A³, Z³, r and R^{c} each have one of the corresponding meanings indicated in Claim 22.

26. Ketene dithioketal of the formula IIc2 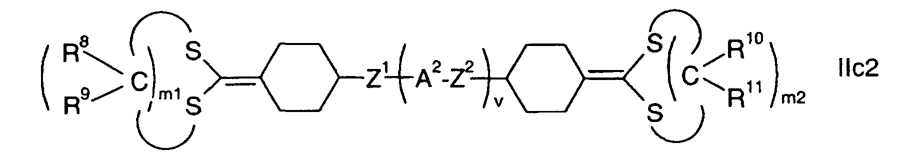 and the corresponding bis(alkylthio)carbenium salt of the formula IIIc2 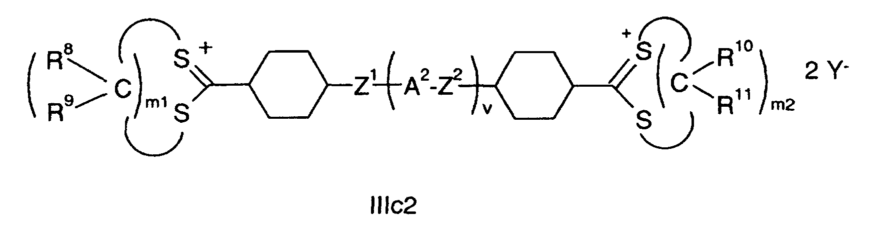 in which
R⁸, R⁹, R¹⁰, R¹¹, m1, m2, Z¹, A², Z² and v have one of the corresponding meanings indicated in Claim 24, and
Y⁻ is a non-coordinating or weakly coordinating anion.

27. Diketone of the formula Ic.1 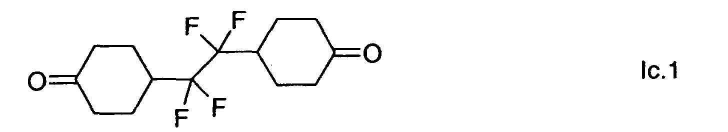 and/or the corresponding ketal of the formula Ic.1.1 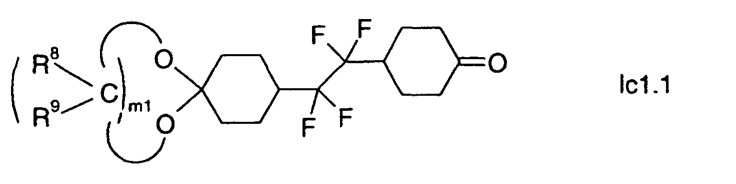 in which
R⁸, R⁹ and m1 have one of the corresponding meanings indicated in Claim 22.

## Revendications

1. Procédé pour la préparation de composés contenant au moins un pont -CF₂-O- dans la molécule, dans lequel
a) un acide est ajouté sur au moins un dithiocétal de cétène,
b) le sel de bis(alkylthio)carbénium résultant est amené à réagir avec au moins un composé qui contient au moins un groupe hydroxyle en présence d'une base,
c) et ensuite, de préférence in situ, le dithioorthoester résultant est soumis à une fluorodésulfurisation oxydative en utilisant un agent de fluoration et un oxydant pour obtenir le composé qui contient au moins un pont -CF₂-O- dans la molécule.

2. Procédé selon la revendication 1, **caractérisé par** un dithiocétal de cétène de la formule II 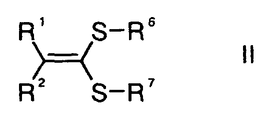 dans laquelle
R¹, R² chacun indépendamment l'un de l'autre, représentent alkyle en chaîne droite, ramifié ou cyclique comportant de 1 à 25 atomes de C, où R¹, R² peuvent être pontés l'un avec l'autre de telle sorte que le groupe 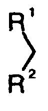 soit un alkyle cyclique comportant de 4 à 8 atomes de C dans l'anneau et/ou dans laquelle un ou plusieurs atomes de H peuvent être remplacés par halogène, -CN, par en outre en option des radicaux alkyle et/ou aryle substitués et/ou dans laquelle un ou plusieurs groupes -CH₂- non adjacents peuvent être remplacés, indépendamment les uns des autres, par -CO-, -O-CO-, -CO-O-, -O-, -S-, -CH=CH-, -C≡C-, -NH- ou -N(CH₃)-, et/ou
aryle, qui peuvent être mono ou polysubstitués par halogène, alkyle et/ou aryle en chaîne droite, ramifiés et/ou cycliques, qui peuvent pareillement être substitués, dans laquelle, en plus, un ou plusieurs groupes CH peuvent être remplacés par N où l'un des radicaux R¹, R² peut représenter H,
R⁶, R⁷ chacun indépendamment l'un de l'autre, représentent alkyle en chaîne droite, ramifié ou cyclique comportant de 1 à 12 atomes de C, où R⁶, R⁷ peuvent être pontés l'un avec l'autre de telle sorte que le groupe 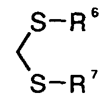 soit un anneau comportant de 4 à 8 éléments et/ou dans laquelle un ou plusieurs atomes de H peuvent être remplacés par halogène, par en outre en option des radicaux alkyle et/ou aryle substitués et/ou dans laquelle un ou plusieurs groupes -CH₂- non adjacents peuvent être remplacés, indépendamment les uns des autres, par -CO-, -O-, -S-, -CH=CH-, -C=C-, -NH- ou -N(CH₃)-, et/ou
aryle, qui peuvent être mono ou polysubstitués par halogène ou alkyle et/ou aryle en chaîne droite, ramifiés et/ou cycliques.

3. Procédé selon la revendication 1 ou 2, **caractérisé en ce que** l'acide à ajouter sur le dithiocétal de cétène est HY, où Y⁻ est un anion de non coordination ou de coordination faible.

4. Procédé selon la revendication 3, **caractérisé en ce que** Y⁻ est un anion de halogénure, de tétrafluoroborate, d'hexafluorophosphate, de perchlorate ou d'alkylcarboxylate ou d'arylcarboxylate ou d'alkylsulfonate ou d'arylsulfonate où , une atome, pluralité d'atomes ou tous les atomes de H dans les groupes alkyle ou aryle peuvent être substitués par fluor ou chlore.

5. Procédé selon l'une quelconque des revendications précédentes, **caractérisé par** un sel de bis(alkylthio)carbénium de la formule III, 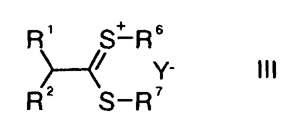 dans laquelle R¹, R², R⁶, R⁷, indépendamment les uns des autres, présentent l'une des significations indiquées dans la revendication 2 et Y⁻ présente l'une des significations indiquées dans la revendication 3 ou 4.

6. Procédé selon l'une quelconque des revendications précédentes, **caractérisé en ce que** le composé contenant au moins un groupe hydroxyle est un alcool alkyle ou aryle de la formule IV R³-OH (IV) dans laquelle le radical alkyle ou aryle R³ peut être substitué selon les souhaits.

7. Procédé selon l'une quelconque des revendications précédentes, **caractérisé par** un dithioorthoester de la formule VI 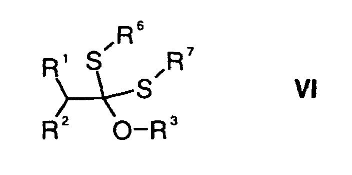 dans laquelle
R¹, R², R⁶, R⁷, indépendamment les uns des autres, présentent l'une des significations indiquées dans la revendication 2 et
R³ présente l'une des significations indiquées dans la revendication 6.

8. Procédé selon l'une quelconque des revendications précédentes, **caractérisé en ce que** l'oxydant est un composé qui libère des équivalents halonium, en particulier est sélectionné parmi le groupe qui est formé par diméthyldibromohydantoïne, N-chlorosuccinimide, N-bromosuccinimide, N-iodosuccinimide, acide dibromoisocyanurique, chlore, brome, SO₂Cl₂, SO₂ClF,
des sels de nitrozonium et de nitronium, des nitrites organiques et inorganiques et chloramine T.

9. Procédé selon l'une quelconque des revendications précédentes, **caractérisé en ce que** l'agent de fluoration est choisi parmi le groupe qui est formé par fluorure d'hydrogène et par des complexes fluorure d'hydrogène/amine aliphatique et aromatique, est choisi en particulier parmi le groupe qui est formé par des complexes pyridine-, triéthylamine-, mélamine-, polyvinylpyridine-fluorure d'hydrogène.

10. Procédé selon l'une quelconque des revendications précédentes, **caractérisé par** un composé qui contient au moins un groupe hydroxyle, de la formule IVa 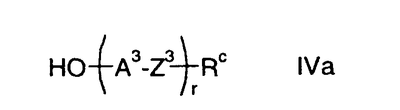 dans laquelle
R^{c} représente H, halogène, -CN, -NCS, -SF₅ ou alkyle comportant de 1 à 18 atomes de C où, en plus, un ou deux groupes -CH₂- non adjacents peuvent être remplacés par -O-, -S-, -CO-, -O-CO-, -CO-O-, -E- et/ou -C≡C-, et/ou, dans laquelle, en plus, un ou plusieurs atomes de H peuvent être remplacés par halogène et/ou -CN,
E représente CR⁴=CR⁵ ou CHR⁴-CHR⁵,
R⁴, R⁵ chacun indépendamment l'un de l'autre, représentent H, alkyle comportant de 1 à 6 atomes de C, F, Cl, Br, CF₃ ou CN,
Z³ est -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- ou une liaison simple,
A³ est 1,4-phénylène où un ou plusieurs groupes CH peuvent être remplacés par N, 1,4-cyclohexylène où un ou deux groupes CH₂ non adjacents peuvent être remplacés par O et/ou S, 1,4-cyclohexénylène, 1,4-bicyclo[2.2.2]octylène, pipéridine-1,4-diyle, naphthalène-2,6-diyle, décahydronaphthalène-2,6-diyle ou 1,2,3,4-tétrahydronaphthalène-2,6-diyle, où un ou plusieurs atomes de H dans ces groupes peuvent être substitués par halogène, -CN et/ou alkyle comportant de 1 à 6 atomes de C où un ou plusieurs atomes de H peuvent être remplacés par halogène ou -CN, et/ou où un ou plusieurs groupes -CH₂- non adjacents peuvent être remplacés indépendamment les uns des autres par -CO-, -O-CO-, -CO-O-, -O-, -S-, -CH=CH-, -C≡C-, -NH- ou -N(CH₃)-, et
r est 0, 1 ou 2
étant entendu que dans le cas où r = 0, R^{c} présente la signification indiquée de alkyle mais où l'atome de C à la position 1 n'a pas été remplacé par un hétéro-atome.

11. Procédé selon l'une quelconque des revendications précédentes, **caractérisé en ce que** le composé contenant au moins un groupe hydroxyle contient deux groupes hydroxyle.

12. Procédé selon la revendication 11, **caractérisé par** un composé contenant deux groupes hydroxyle, de la formule IVd 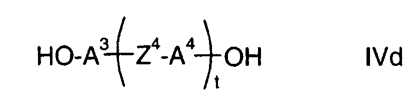 dans laquelle
A³ présente l'une des significations indiquées selon la revendication 10,
A⁴ présente l'une des significations indiquées pour A³, et
Z⁴ présente l'une des significations indiquées pour Z³ selon la revendication 10, et
t est 0, 1 ou 2.

13. Procédé selon l'une des revendications précédentes, **caractérisé en ce que** le dithiocétal de cétène est obtenu à partir d'un composé de carbonyle.

14. Procédé selon la revendication 13, **caractérisé par** un composé de carbonyle de la formule I 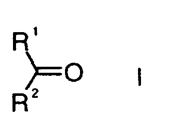 dans laquelle R¹ et R², indépendamment l'un de l'autre, présentent l'une des significations indiquées selon la revendication 2.

15. Procédé selon la revendication 13 ou 14, **caractérisé par** un composé de carbonyle de la formule la 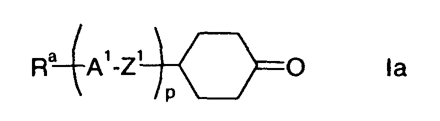 dans laquelle
R^{a} représente H, halogène, -CN ou alkyle comportant de 1 à 18 atomes de C où, en plus, un ou deux groupes -CH₂- non adjacents peuvent être remplacés par -O-, -S-, -CO-, -O-CO-, -CO-O-, -E- et/ou -C≡C-, et/ou, où en plus, un ou plusieurs atomes de H peuvent être remplacés par halogène et/ou -CN,
E représente CR⁴=CR⁵ ou CHR⁴-CHR⁵,
R⁴, R⁵ chacun indépendamment l'un de l'autre, représentent H, alkyle comportant de 1 à 6 atomes de C, F, Cl, Br, CF₃ ou CN;et
Z¹ est -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- ou une liaison simple, et
A¹ est 1,4-phénylène où un ou plusieurs groupes CH peuvent être remplacés par N, 1,4-cyclohexylène où un ou deux groupes CH₂ non adjacents peuvent être remplacés par O et/ou S, 1,4-cyclohexénylène, 1,4-bicyclo[2.2.2]octylène, pipéridine-1,4-diyle, naphthalène-2,6-diyle, décahydronaphthalène-2,6-diyle ou 1,2,3,4-tétrahydronaphthalène-2,6-diyle, où un ou plusieurs atomes de H dans ces groupes peuvent être substitués par halogène, -CN et/ou alkyle comportant de 1 à 6 atomes de C, où un ou plusieurs atomes de H peuvent être remplacés par halogène ou -CN et/ou un ou plusieurs groupes -CH₂- non adjacents peuvent être remplacés, indépendamment les uns des autres, par -CO-, -O-CO-, -CO-O-, -O-, -S-, -CH=CH-, -C≡C-, -NH- ou -N(CH₃)-, et
p est 0, 1 ou 2.

16. Procédé selon l'une des revendications précédentes, **caractérisé en ce que** le composé de carbonyle contient deux groupes carbonyle ou plus.

17. Procédé selon la revendication 16, **caractérisé en ce que** le composé de carbonyle qui contient deux groupes carbonyle ou plus est un cyclohexanedione de la formule Ib 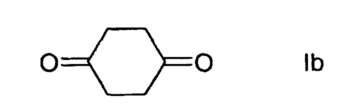 ou un composé de la formule Ic 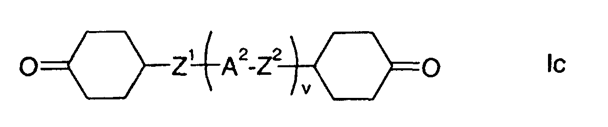 dans laquelle
Z¹, Z² chacun indépendamment l'un de l'autre, présentent l'une des significations indiquées pour Z¹ selon la revendication 15;
A² présente l'une des significations indiquées pour A¹ selon la revendication 15, et
v est 0, 1 ou 2.

18. Procédé selon la revendication 16 ou 17, **caractérisé en ce qu'**un ou plusieurs groupes carbonyle du composé de carbonyle sont protégés en tant que cétal avant la conversion selon le dithiocétal de cétène correspondant, au moins un groupe carbonyle restant non protégé pour une conversion selon le dithiocétal de cétène.

19. Procédé selon l'une des revendications 13 à 18, **caractérisé en ce que** le dithiocétal de cétène est obtenu à partir du composé de carbonyle par réaction avec du 2-silyl-1,3-dithiane substitué ou non substitué.

20. Dithiocétal de cétène de la formule IIa 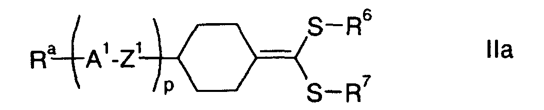 et/ou le sel de bis(alkylthio)carbénium correspondant de la formule IIIa 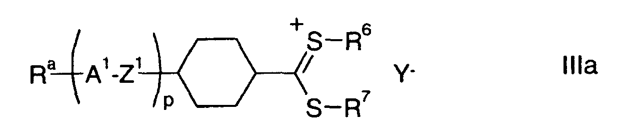 dans lesquelles
R^{a} représente H, halogène, -CN ou alkyle comportant de 1 à 18 atomes de C où, en plus, un ou deux groupes -CH₂- non adjacents peuvent être remplacés par -O-, -S-, -CO-, -O-CO-, -CO-O-, -E- et/ou -C≡C-, et/ou, où, en plus, un ou plusieurs atomes de H peuvent être remplacés par halogène et/ou CN,
E représente CR⁴=CR⁵ ou CHR⁴-CHR⁵,
R⁴, R⁵ chacun indépendamment l'un de l'autre, représentent H, alkyle comportant de 1 à 6 atomes de C, F, Cl, Br, CF₃ ou CN ; et
Z¹ est -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- ou une liaison simple,
A¹ est 1,4-phénylène où un ou plusieurs groupes CH peuvent être remplacés par N, 1,4-cyclohexylène où un ou deux groupes CH₂ non adjacents peuvent être remplacés par O et/ou S, 1,4-cyclohexénylène, 1,4-bicyclo[2.2.2]octylène, pipéridine-1,4-diyle, naphthalène-2,6-diyle, décahydronaphthalène-2,6-diyle ou 1,2,3,4-tétrahydronaphthalène-2,6-diyle, où un ou plusieurs atomes de H dans ces groupes peuvent être substitués par halogène, -CN et/ou alkyle comportant de 1 à 6 atomes de C, où un ou plusieurs atomes de H peuvent être remplacés par halogène ou -CN et/ou où un ou plusieurs groupes -CH₂- non adjacents peuvent être remplacés, indépendamment les uns des autres, par -CO-, -O-CO-, -CO-O-, -O-, -S-, -CH=CH-, -C≡C-, -NH- ou -N(CH₃)-,
p est 0, 1 ou 2, et
R⁶, R⁷ indépendamment l'un de l'autre, représentent alkyle en chaîne droite, ramifié ou cyclique comportant de 1 à 12 atomes de C, où un ou plusieurs atomes de H peuvent être remplacés par halogène, par en outre en option des radicaux alkyle et/ou aryle substitués et/ou où un ou plusieurs groupes -CH₂- non adjacents peuvent être remplacés, indépendamment les uns des autres, par -CO-, -O-, -S-, -CH=CH-, -C≡C-, -NH- ou -N(CH₃)-, et/ou
aryle, qui peuvent être mono ou polysubstitués par halogène, alkyle et/ou aryle en chaîne droite, ramifiés et/ou cycliques, et,
Y⁻ est un anion de non coordination ou de coordination faible.

21. Dithiocétal de cétène de la formule IIb1 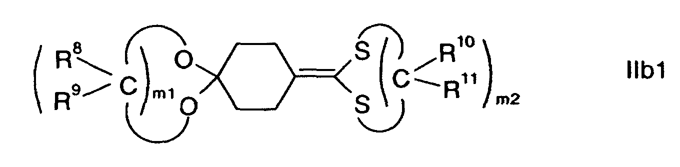 et/ou le sel de bis(alkylthio)carbénium correspondant de la formule IIIb1 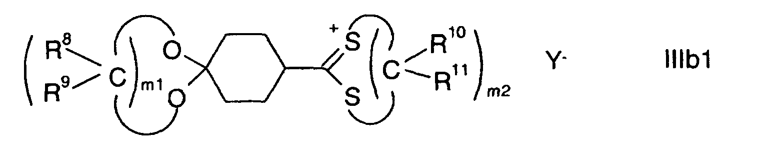 dans lesquelles
R⁸, R⁹, R¹⁰, R¹¹ chacun indépendamment l'un de l'autre, représentent H ou un groupe alkyle ou alkényle substitué ou non substitué comportant de 1 à 6 atomes de C où, dans chaque cas, un groupe pris parmi 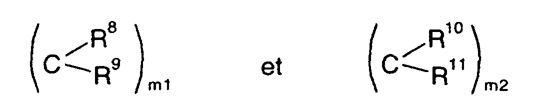 ou les deux groupes peuvent former un groupe cycloalkyle ou aryle, et
m1, m2, sont 2, 3 ou 4, et
Y⁻ est un anion de non coordination ou de coordination faible.

22. Composé de la formule Vb 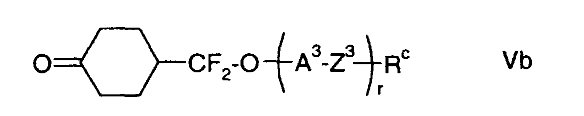 et/ou le cétal correspondant de la formule Vb1 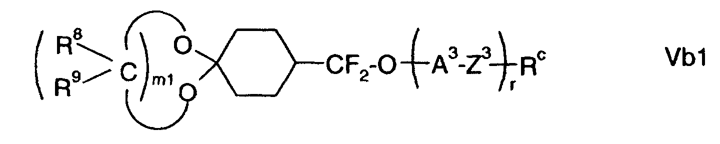 dans lesquelles
R⁸, R⁹ et m1 présentent l'une des significations correspondantes indiquées selon la revendication 21, et
R^{c}, représente H, halogène, -CN, -NCS, -SF₅ ou alkyle comportant de 1 à 18 atomes de C où, en plus, un ou deux groupes -CH₂- non adjacents peuvent être remplacés par -O-, -S-, -CO-, -O-CO-, -CO-O-, -E- et/ou -C≡C-, et/ou où, en plus, un ou plusieurs atomes de H peuvent être remplacés par halogène et/ou -CN,
E représente CR⁴=CR⁵ ou CHR⁴-CHR⁵,
R⁴, R⁵ chacun indépendamment l'un de l'autre, représentent H, alkyle comportant de 1 à 6 atomes de C, F, Cl, Br, CF₃ ou CN, et
Z³ est -O-CO-, -CO-O-, -C₂H₄-, -CH₂-CF₂-, -CF₂-CH₂-, -CF₂-CF₂-, -CH₂-O-, -CF₂-O-, -O-CH₂-, -O-CF₂-, -CH=CH-, -CF=CH-, -CH=CF-, -CF=CF-, -C≡C- ou une liaison simple, et
A³ est 1,4-phénylène où un ou plusieurs groupes CH peuvent être remplacés par N, 1,4-cyclohexylène où un ou deux groupes CH₂ non adjacents peuvent être remplacés par O et/ou S, 1,4-cyclohexénylène, 1,4-bicyclo[2.2.2]octylène, pipéridine-1,4-diyle, naphthalène-2,6-diyle, décahydronaphthalène-2,6-diyle ou 1,2,3,4-tétrahydronaphthalène-2,6-diyle, où un ou plusieurs atomes de H dans ces groupes peuvent être substitués par halogène, -CN et/ou alkyle comportant de 1 à 6 atomes de C où un ou plusieurs atomes de H peuvent être remplacés par halogène ou -CN, et/ou où un ou plusieurs groupes -CH₂- non adjacents peuvent être remplacés indépendamment les uns des autres par -CO-, -O-CO-, -CO-O-, -O-, -S-, -CH=CH-, -C≡C-, -NH- ou -N(CH₃)-, et
r est 0, 1 ou 2,
étant entendu que dans le cas où r = 0, R^{c} présente la signification indiquée de alkyle mais où l'atome de C à la position 1 n'a pas été remplacé par un hétéro-atome.

23. Dithiocétal de cétène de la formule IIb2 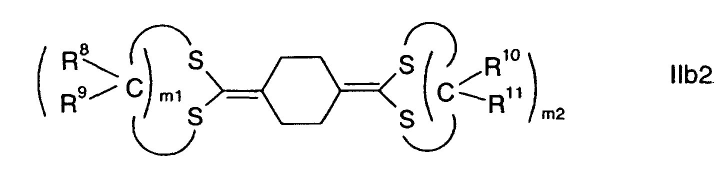 et/ou le sel de bis(alkylthio)carbénium correspondant de la formule IIIb2 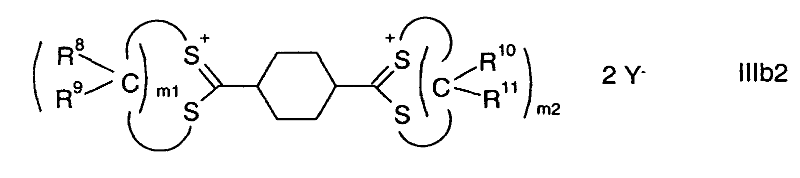 dans lesquelles
R⁸, R⁹, R¹⁰, R¹¹, m1 et m2 présentent chacun l'une des significations correspondantes indiquées selon la revendication 21, et
Y⁻ est un anion de non coordination ou de coordination faible.

24. Dithiocétal de cétène de la formule IIc1 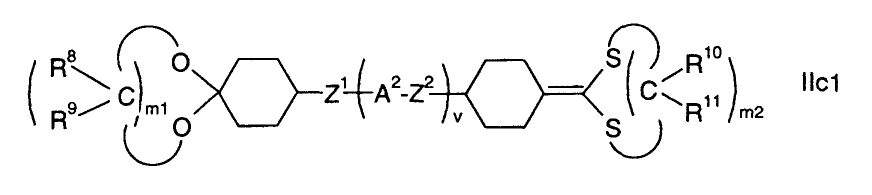 et/ou le sel de bis(alkylthio)carbénium correspondant de la formule IIIc1 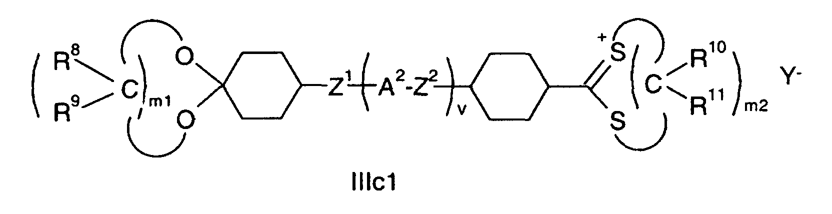 dans lesquelles
R⁸, R⁹, R¹⁰, R¹¹, m1 et m2 présentent l'une des significations correspondantes indiquées selon la revendication 21,
Z¹ présente l'une des significations correspondantes indiquées selon la revendication 20, et
A² et Z² présentent l'une des significations correspondant à A¹ et Z¹, comme respectivement indiqué selon la revendication 20,
v est 0, 1 ou 2, et
Y⁻ est un anion de non coordination ou de coordination faible.

25. Composé de la formule Vc 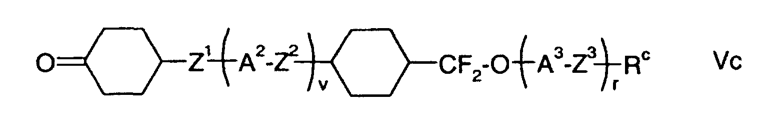 et/ou le cétal correspondant de la formule Vc1 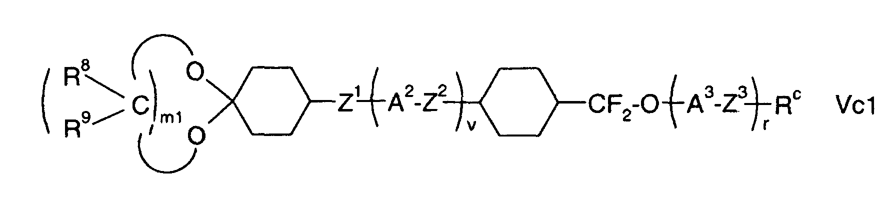 dans lesquelles
R⁸, R⁹, m1, Z¹, A², Z² et v présentent l'une des significations correspondantes indiquées selon la revendication 24, et
A³, Z³, r et R^{c}, présentent chacun l'une des significations correspondantes indiquées selon la revendication 22.

26. Dithiocétal de cétène de la formule IIc2 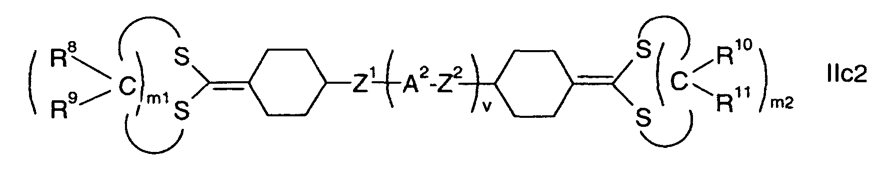 et le sel de bis(alkylthio)carbénium correspondant de la formule IIIc2 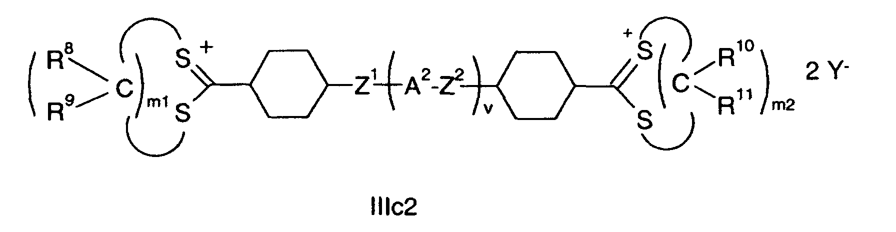 dans lesquelles
R⁸, R⁹, R¹⁰, R¹¹, m1, m2, Z¹, A², Z² et v présentent l'une des significations correspondantes indiquées selon la revendication 24, et
Y⁻ est un anion de non coordination ou de coordination faible.

27. Dicétone de la formule Ic.1 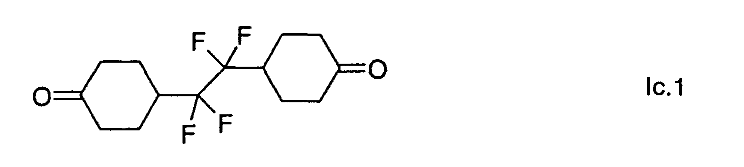 et/ou le cétal correspondant de la formule Ic1.1 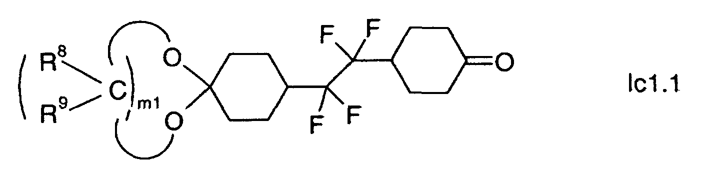 dans lesquelles
R⁸, R⁹ et m1 présentent l'une des significations correspondantes indiquées selon la revendication 22.
